# EUROPEAN PATENT SPECIFICATION

(11) **EP 4 372 378 B1**
(45) Date of publication and mention of the grant of the patent: **18.03.2026**
(21) Application number: 22837658.8
(22) Date of filing: 05.07.2022
(51) Int. Cl.: C07K 14/005, C12N 15/85, G01N 33/576, C07K 16/10

(54) **METHOD FOR DETECTING HEPATITIS E VIRUS INFECTION, AND ANTIGEN POLYPEPTIDE AND KIT FOR DETECTING HEPATITIS E VIRUS INFECTION**
VERFAHREN ZUM NACHWEIS EINER HEPATITIS-E-VIRUS-INFEKTION SOWIE ANTIGENPOLYPEPTID UND KIT ZUM NACHWEIS EINER HEPATITIS-E-VIRUS-INFEKTION
MÉTHODE DE DÉTECTION D'UNE INFECTION PAR LE VIRUS DE L'HÉPATITE E, ET POLYPEPTIDE ANTIGÉNIQUE ET KIT DE DÉTECTION D'UNE INFECTION PAR LE VIRUS DE L'HÉPATITE E

(30) Priority: 06.07.2021 JP 2021112045
(43) Date of publication of application: 22.05.2024
(73) Proprietor: Advanced Life Science Institute, Inc., Tokyo 192-0031 (JP)
(72) Inventor: MAKI, Noboru, Hachioji-shi, Tokyo 192-0031 (JP); MORI, Kenichi, Hachioji-shi, Tokyo 192-0031 (JP); YAGI, Shintaro, Hachioji-shi, Tokyo 192-0031 (JP)
(74) Representative: Müller-Boré & Partner Patentanwälte PartG mbB
(86) International application number: PCT/JP2022/026662
(87) International publication number: WO 2023/282243

(56) References cited:
- EP-A1- 1 452 541
- CN-A- 1 793 929
- CN-A- 1 793 929
- CN-A- 101 062 941
- CN-A- 101 880 318
- CN-A- 102 276 700
- CN-A- 104 031 144
- CN-A- 109 765 374
- JP-A- 2004 500 041
- JP-A- 2004 525 613
- JP-A- H0 797 398
- JP-A- H0 797 398
- US-B2- 8 524 868
- PAN JIN-SHUN ET AL: "Application of Truncated Immunodominant Polypeptide from Hepatitis E Virus (HEV) ORF2 in an Assay To Exclude Nonspecific Binding in Detecting Anti-HEV Immunoglobulin M", JOURNAL OF CLINICAL MICROBIOLOGY, vol. 48, no. 3, 1 March 2010 (2010-03-01), US, pages 779 - 784, XP093022542, ISSN: 0095-1137, DOI: 10.1128/JCM.01671-09
- PAN JIN-SHUN, ZHANG KUI, ZHOU JINYONG, WU CHAO, ZHUANG HUI, ZHOU YI-HUA: "Application of Truncated Immunodominant Polypeptide from Hepatitis E Virus (HEV) ORF2 in an Assay To Exclude Nonspecific Binding in Detecting Anti-HEV Immunoglobulin M", JOURNAL OF CLINICAL MICROBIOLOGY, AMERICAN SOCIETY FOR MICROBIOLOGY, US, vol. 48, no. 3, 1 March 2010 (2010-03-01), US , pages 779 - 784, XP093022542, ISSN: 0095-1137, DOI: 10.1128/JCM.01671-09
- ZAHMANOVA GERGANA, MAZALOVSKA MILENA, TAKOVA KATERINA, TONEVA VALENTINA, MINKOV IVAN, PEYRET HADRIEN, LOMONOSSOFF GEORGE: "Efficient Production of Chimeric Hepatitis B Virus-Like Particles Bearing an Epitope of Hepatitis E Virus Capsid by Transient Expression in Nicotiana benthamiana", LIFE, vol. 11, no. 1, pages 64, XP093022543, DOI: 10.3390/life11010064
- BAI CHONGZHI; CAI JIANPIAO; HAN PENGCHENG; QI JIANXUN; YUEN KWOK-YUNG; WANG QIHUI: "The crystal structure of the emerging human-infecting hepatitis E virus E2s protein", BIOCHEMICAL AND BIOPHYSICAL RESEARCH COMMUNICATIONS, ELSEVIER, AMSTERDAM NL, vol. 532, no. 1, 18 August 2020 (2020-08-18), Amsterdam NL , pages 25 - 31, XP086270577, ISSN: 0006-291X, DOI: 10.1016/j.bbrc.2020.07.074
- ANDERSON D. A., ET AL.: "ELISA FOR IGG-CLASS ANTIBODY TO HEPATITIS E VIRUS BASED ON A HIGHLYCONSERVED, CONFORMATIONAL EPITOPE EXPRESSED IN ESCHERICHIA COLI.", JOURNAL OF VIROLOGICAL METHODS, ELSEVIER BV, NL, vol. 81., no. 1-2, 1 August 1999 (1999-08-01), NL , pages 131 - 142., XP000942370, ISSN: 0166-0934, DOI: 10.1016/S0166-0934(99)00069-5
- DESHMUKH, T.M. LOLE, K.S. TRIPATHY, A.S. ARANKALLE, V.A.: "Immunogenicity of candidate hepatitis E virus DNA vaccine expressing complete and truncated ORF2 in mice", VACCINE, ELSEVIER, AMSTERDAM, NL, vol. 25, no. 22, 6 May 2007 (2007-05-06), AMSTERDAM, NL , pages 4350 - 4360, XP022062912, ISSN: 0264-410X, DOI: 10.1016/j.vaccine.2007.03.040
- ZHANG JUN ET AL: "Analysis of hepatitis E virus neutralization sites using monoclonal antibodies directed against a virus capsid protein.", VACCINE, ELSEVIER, AMSTERDAM, NL, vol. 23, no. 22, 22 April 2005 (2005-04-22), AMSTERDAM, NL , pages 2881 - 2892, XP002327835, ISSN: 0264-410X, DOI: 10.1016/j.vaccine.2004.11.065
- TANG XUHUA, CHUNYAN YANG, YING GU, CUILING SONG, XIAO ZHANG, YINGBIN WANG, JUN ZHANG, CHOY LEONG HEW, SHAOWEI LI, NINGSHAO XIA, J.: "Structural basis for the neutralization and genotype specificity of hepatitis E virus", PROCEEDINGS OF THE NATIONAL ACADEMY OF SCIENCES OF THE UNITED STATES OF AMERICA, vol. 108, no. 25, 3 June 2011 (2011-06-03), pages 10266 - 10271, XP093022549, DOI: 10.1073/pnas.1101309108
- HIROYUKI OKU, YUSUKE HORI, MINORU YAMAJI, YUKO OKU: "Synthetic Peptides Designed from Hepatitis E Virus Capsid Protein and Their Reactivity Against Sera from Wild Boars in Gunma Prefecture, Japan", PEPTIDE SCIENCE 2020 : PROCEEDINGS OF THE 57TH JAPANESE PEPTIDE SYMPOSIUM; NOVEMBER 9-11, 2020, JAPANESE PEPTIDE SOCIETY, JP, vol. 57, 28 February 2021 (2021-02-28) - 11 November 2020 (2020-11-11), JP, pages 61 - 62, XP009542872
- BOUMAIZA MOHAMED, KHALED TRABELSI, ZEINEB CHOUCHA, INES AKROUTI, SERENA LEONE, DELIA PICONE, HÉLA KALLEL: "Production and characterization of a fusion form of hepatitis E virus tORF2 capsid protein in Escherichia coli", PREPARATIVE BIOCHEMISTRY & BIOTECHNOLOGY, vol. 51, no. 6, 23 October 2020 (2020-10-23), pages 562 - 569, XP093022552, DOI: 10.1080/10826068.2020.1836656

## Description

### [Technical Field]

The present invention relates to a method for detecting hepatitis E virus infection, an antigen polypeptide and kit for detecting hepatitis E virus infection used therefor.

### [Background Art]

Hepatitis E is an acute or fulminant hepatitis caused by the hepatitis E virus (HEV), and its mortality rate is said to be about 10 times that of hepatitis A. There have also been reports that the mortality rate in pregnant women reaches 20%. Hepatitis E is known to sporadically occur mainly in India, Asia, Africa, and Central and South America. However, in other regions, with the diversification of diet in recent years, there have been reported cases where the onset is strongly suspected due to the oral intake of food contaminated with HEV (for example, meat such as pork, wild boar, and deer) without heating. Additionally, there are increasingly visible instances of infection after blood transfusion and reactivation of hepatitis. Therefore, the demand for tests to investigate the presence or absence of HEV infection in subjects is increasing every year.

Traditionally, the detection of HEV infection in subjects was carried out by detecting the HEV gene in the subject's serum using the PCR method. However, the HEV gene in the serum can only be detected for a limited period of approximately two weeks to one month after HEV infection, making detection outside of this period challenging.

Furthermore, the concentration of HEV secreted by infected humans or animals is low, making it also difficult to directly detect HEV using anti-hepatitis E virus antibodies (anti-HEV antibodies). Therefore, the mainstream method currently applied clinically is an indirect method of detecting HEV infection by detecting anti-HEV antibodies in the subject using antigen polypeptides or recombinant antigens synthesized based on HEV. For example, anti-HEV antibodies in the IgA class or IgM class persist for about two to five months after hepatitis E virus infection.

Such methods and reagents used therefor include, for example, commercially available kits for detecting IgA class anti-HEV antibodies using a two-step ELISA with a plate immobilized with recombinant HEV antigen polypeptide and labeled anti-human IgA antibody. NPL 1 describes the detection of anti-HEV antibodies using amino acids 111 to 660 encoded by the ORF2 of the HEV genotype 4 gene (DDBJ/GenBank/EMBL Accession No.: AB082545) as recombinant HEV antigen polypeptide.

Furthermore, commercially available kits for detecting IgM class anti-HEV antibodies using a two-step ELISA with a plate immobilized with labeled recombinant HEV antigen polypeptide and anti-human IgM antibody are also on the market. International Application Japanese-Phase Publication No. 2004-525613 (PTL 1) describes a kit for diagnosing HEV infection, which includes polypeptide fragments 394 to 606 in the sequence equivalent to the amino acid sequence encoded by the ORF2 of the HEV genotype 1 gene (DDBJ Accession No.: D11092). CN1793929 discloses polypeptides of ORF2 of HEV to detect HEV genotype specific antibodies.

### [Citation List]

### [Patent Literature]

[PTL 1] International Application Japanese-Phase Publication No. 2004-525613

### [Non Patent Literature]

[NPL 1] Mizuo H. et al., J. Clin. Microbiol., Vol. 40, No.9, 2002, p. 3209-3218

### [Summary of Invention]

### [Technical Problem]

However, upon further investigation by the present inventors into the kits currently used for detecting Hepatitis E virus infections in clinical applications in Japan, it was found that many samples were incapable of detection by the conventional kits, indicating that the detection sensitivity for anti-HEV antibody using these kits is still not sufficient.

The present invention has been made in view of the above problems identified by the present inventors, and aims to provide a method for detecting hepatitis E virus infections with higher sensitivity than conventional methods, allowing for detection of anti-hepatitis E virus antibodies, and an antigen polypeptide and kit for detecting hepatitis E virus infections used therefor.

### [Solution to Problem]

The present inventors have discovered a method for indirectly detecting HEV infection, specifically a method for detecting HEV infection by detecting anti-HEV antibodies through an HEV antigen. In this method, the E2s domain, positioned on the C-terminus side of the amino acids encoded by ORF2, which encodes the HEV capsid protein, is particularly employed as the HEV antigen polypeptide. Further, by shortening the length of this polypeptide to 200 residues or less. Thereby, it was found that it was possible to achieve high-sensitivity detection even for samples that could not be detected with conventional kits, that is, kits which use amino acids 111 to 660 encoded by the ORF2 of the HEV genotype 4 gene (DDBJ/GenBank/EMBL Accession No.: AB082545) as the HEV antigen polypeptide, or kits which use amino acids 394 to 606 encoded by the ORF2 of the HEV genotype 1 gene (DDBJ Accession No.: D11092) as the HEV antigen polypeptide. This discovery has led to the completion of the present invention. The embodiments of the present invention obtained through such findings are as follows.

A method for detecting hepatitis E virus infection, comprising a detection step of detecting an anti-hepatitis E virus antibody in a sample by using, as an antigen polypeptide, a dimer consisting of any one polypeptide selected from the group consisting of the following (3a) to (3c), and any one polypeptide selected from the group consisting of the following (1a) to (1c) (2a) to (2c), and (4a) to (4c):
(1a) a polypeptide consisting of an amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(1b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(1c) a polypeptide consisting of an amino acid sequence having 80% or more homology with the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(2a) a polypeptide consisting of an amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(2b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(2c) a polypeptide consisting of an amino acid sequence having 80% or more homology with the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(3a) a polypeptide consisting of an amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(3b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(3c) a polypeptide consisting of an amino acid sequence having 80% or more homology with the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(4a) a polypeptide consisting of an amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4;
(4b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4; and
(4c) a polypeptide consisting of an amino acid sequence having 80% or more homology with the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4.

[2] The method for detecting hepatitis E virus infection according to [1] wherein an isotype of the anti-hepatitis E virus antibody is IgM.

[3] The method for detecting hepatitis E virus infection according to [1] wherein
the detection step comprises a step of bringing the sample into contact with a capture body and a labeled body, and
the capture body comprises a water insoluble carrier and a probe molecule capable of binding to the anti-hepatitis E virus antibody, and
the labeled body comprises a labeling substance and the antigen polypeptide.

[4] An antigen polypeptide for detecting hepatitis E virus infection according to the method for detecting hepatitis E virus infection according to [1] which is a dimer consisting of any one polypeptide selected from the group consisting of the following (3a) to (3c), and any one polypeptide selected from the group consisting of the following (1a) to (1c), (2a) to (2c), and (4a) to (4c):
(1a) a polypeptide consisting of an amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(1b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(1c) a polypeptide consisting of an amino acid sequence having 80% or more homology with the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(2a) a polypeptide consisting of an amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(2b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(2c) a polypeptide consisting of an amino acid sequence having 80% or more homology with the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(3a) a polypeptide consisting of an amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(3b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(3c) a polypeptide consisting of an amino acid sequence having 80% or more homology with the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(4a) a polypeptide consisting of an amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4;
(4b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4; and
(4c) a polypeptide consisting of an amino acid sequence having 80% or more homology with the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4.

[5] The antigen polypeptide according to [4], which is a labeled body labeled with a labeling substance.

[6] A kit for detecting hepatitis E virus infection according to the method for detecting hepatitis E virus infection according to [1] comprising the antigen polypeptide according tc any one of [4] or [5].

[7] The kit for detecting hepatitis E virus infection according to [6], further comprising a capture body comprising a water insoluble carrier and a probe molecule capable of binding to an anti-hepatitis E virus antibody.

### [Advantageous Effects of Invention]

The present invention makes it possible to provide a method for detecting hepatitis E virus infections with higher sensitivity than conventional methods, allowing for detection of anti-hepatitis E virus antibodies, and an antigen polypeptide and kit for detecting hepatitis E virus infections used therefor.

### [Brief Description of Drawings]

[Fig. 1] Fig. 1 shows the amino acid sequences of genotypes 1 to 4 (Gen1 to Gen4), set forth in SEQ ID NOs: 1 to 4.
[Fig. 2] Fig. 2 is an electrophoretic photograph obtained by performing SDS-polyacrylamide gel electrophoresis on solutions or precipitates obtained at each purification stage of Hybrid-E2s (G3/G1-E2s).
[Fig. 3A] Fig. 3A is an electrophoretic photograph obtained by performing SDS-polyacrylamide gel electrophoresis on solutions or precipitates obtained at each purification stage of Hybrid-E2s (G3/G4-E2s).
[Fig. 3B] Fig. 3B is an electrophoretic photograph obtained by performing SDS-polyacrylamide gel electrophoresis on solutions or precipitates obtained at each purification stage of Hybrid-E2s (G3/G4-E2s).

### [Description of Embodiments]

Hereinafter, the present invention will be described in detail with reference to preferred embodiments thereof.

### <Method for Detecting Hepatitis E Virus Infection>

The method of the present invention is detailed in the appended claims. Embodiments which do not fall under the scope of the claims are described herein to allow better understanding of the invention.

### [Hepatitis E Virus]

In the present invention, the presence of the hepatitis E virus is indirectly detected by detecting antibodies in the sample against the hepatitis E virus, namely, anti-hepatitis E virus antibodies. More specifically, the method detects the presence or absence of hepatitis E virus infection in the sample, preferably the subject from whom the sample originates. The hepatitis E virus (occasionally referred to as "HEV" in the present specification) is a small spherical particle not covered by an envelope (diameter: 27 to 34 nm) and currently, four genotypes (Genotype 1 to Genotype 4) are known. Its genome is a single-stranded RNA of approximately 7200 bases and possesses 3 ORFs (open reading frames: ORF1, ORF2, and ORF3). ORF1 encodes non-structural proteins such as helicase and RNA polymerase, ORF2 encodes capsid proteins, and ORF3 encodes a phosphorylated protein composed of 113 or 114 amino acid residues. Among these, ORF2 typically encodes a polypeptide composed of an amino acid sequence of 660 residues, with amino acids 129 to 606 of the polypeptide constituting the capsid protein, which includes, from the N-terminus side thereof, the S domain forming the virus shell (typically, amino acids 129 to 319); the M domain forming 2, 3, or 5-mer (M: typically, amino acids 320 to 455); and the P domain forming the protrusions (P: typically, amino acids 456 to 606). In addition, it has been reported that the E2s domain, typically composed of amino acids 459 to 606 of the amino acid sequence encoded by ORF2, forms a dimer and plays a critical role in host recognition by the virus (Li, S. et al., PLoS Pathog. 5, e1000537, 2009). Note that in the present invention, when indicating a number as ordinal in amino acid sequences, it is understood as the number of amino acid residues from the N-terminus side.

### [Anti-Hepatitis E Virus Antibody]

In the present invention, the anti-hepatitis E virus antibody (occasionally referred to as "anti-HEV antibody" in the present specification) is an antibody that can specifically bind to the hepatitis E virus, more specifically an antibody produced within the body of the subject from whom the sample originates. As long as it can recognize the following antigen polypeptide, the anti-HEV antibody according to the present invention does not have to be a complete antibody; it may even be an antibody fragment.

The isotype of the anti-HEV antibody according to the present invention is not particularly limited, and may be any of IgG, IgM, IgA, IgE, or IgD, or a combination of two or more thereof. Also, any subclass may be used for each of the aforementioned isotypes. For example, when humans are infected with HEV, IgM appears first in the serum, followed by IgA, lasting for several months (up to about five months). Thus, detecting these targets allows for the detection of infections at the early stages. Also, IgG appears slightly later and lasts for a longer period, making it possible to detect past HEV infections. Among these, from the viewpoint of earlier detection when using the sandwich method described later (such as sandwich ELISA), IgM is preferable, and from the viewpoint of enabling more detailed analysis such as identifying the time of infection, a combination of IgM and IgG is preferred, but the isotype is not limited to these.

### [Subject]

In the present invention, "subject" refers to the individual from whom the sample for the method for detecting hepatitis E virus infection according to the present invention originates. It also refers to the target to whom the following method for testing hepatitis E virus according to the present invention is applied. Such subjects may include humans or non-human mammals (such as pigs, wild boars, cattle, and deer), preferably humans. There are no specific restrictions on the subject according to the present invention, and it may be a healthy individual not infected with HEV and not developing hepatitis E, or an individual who is infected with HEV but has not developed hepatitis E. Furthermore, it may also be a patient who is already known to be infected with HEV or who has previously developed hepatitis E, aiming to identify the onset period and the like.

### [Sample]

In the present invention, the term "sample" refers to the target of the method for detecting hepatitis E virus infection of the present invention, and there are no particular restrictions as long as the sample is one in which the above-described anti-HEV antibodies may exist. Typically, examples thereof include blood samples such as serum, plasma, and whole blood taken from the target such as the above-described subject; body fluid samples other than blood, such as urine, feces, sputum, saliva, sweat, cerebrospinal fluid, digestive fluid, semen, lymph, and ascites; mucosal samples such as oral mucosa, pharyngeal mucosa, and intestinal mucosa; and various biopsy samples. Among these, blood samples are preferable as samples according to the present invention, with serum or plasma being more preferable.

These samples may be diluted or suspended in a diluent as necessary. Examples of the diluents include a buffer solution such as phosphate buffer solution, Tris buffer solution, Good's buffer solution, borate buffer solution, acetate buffer solution, citrate buffer solution, glycine buffer solution, succinate buffer solution, phthalate buffer solution, and the like. Also, as a sample according to the present invention, it can be appropriately pretreated as necessary. Examples of those pretreatments include procedures such as pulverization, freezing, heating, concentration, fractionation, and desalination; addition of pH adjusters, stabilizers, preservatives, antiseptics, and surfactants; purification procedures, etc. These can be used individually or in any combination of two or more.

### [Antigen Polypeptide]

The method of the present invention comprises a detection step of detecting anti-HEV antibodies in a sample by using, as an antigen polypeptide, a dimer consisting of any one polypeptide selected from the group consisting of the claimed (3a) to (3c), and any one polypeptide selected from the group consisting of the claimed (1a) to (1c), (2a) to (2c), and (4a) to (4c). In the present invention, "detection" includes, in addition to detecting the presence or absence and amount of anti-HEV antibodies in a sample as a signal, quantifying or semi-quantifying the detected anti-HEV antibodies according to the signal intensity.

The antigen polypeptide used for the detection of anti-HEV antibodies is typically (a) a polypeptide consisting of an amino acid sequence which contains amino acids located at position 28 to 175 in the amino acid sequence set forth in any one of SEQ ID NOs: 1 to 4 and which has a full length of 200 residues or less that are contained in the amino acid sequence set forth in that sequence number, namely, at least one polypeptide selected from the group consisting of
(1a) a polypeptide consisting of an amino acid sequence which contains amino acids located at position 28 to 175 in the amino acid sequence set forth in SEQ ID NO: 1, and whose full length is 200 residues or less that are contained in the amino acid sequence set forth in SEQ ID NO: 1;
(2a) a polypeptide consisting of an amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(3a) a polypeptide consisting of an amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3; and (4a) a polypeptide consisting of an amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4 (hereinafter occasionally referred to as "antigen polypeptide (a)").

The amino acid sequence set forth in SEQ ID NO: 1 corresponds to the amino acid sequence at positions 432 to 660 of the ORF2 of HEV genotype 1 (DDBJ Accession No.: D11092). The amino acid sequence set forth in SEQ ID NO: 2 corresponds to the amino acid sequence at positions 432 to 660 of the ORF2 of HEV genotype 2 (DDBJ Accession No.: M74506). The amino acid sequence set forth in SEQ ID NO: 3 corresponds to the amino acid sequence at positions 432 to 660 of the ORF2 of HEV genotype 3 (GenBank Accession No.: AB481229). The amino acid sequence set forth in SEQ ID NO: 4 corresponds to the amino acid sequence at positions 432 to 660 of the ORF2 of HEV genotype 4 (GenBank Accession No.: AB481227).

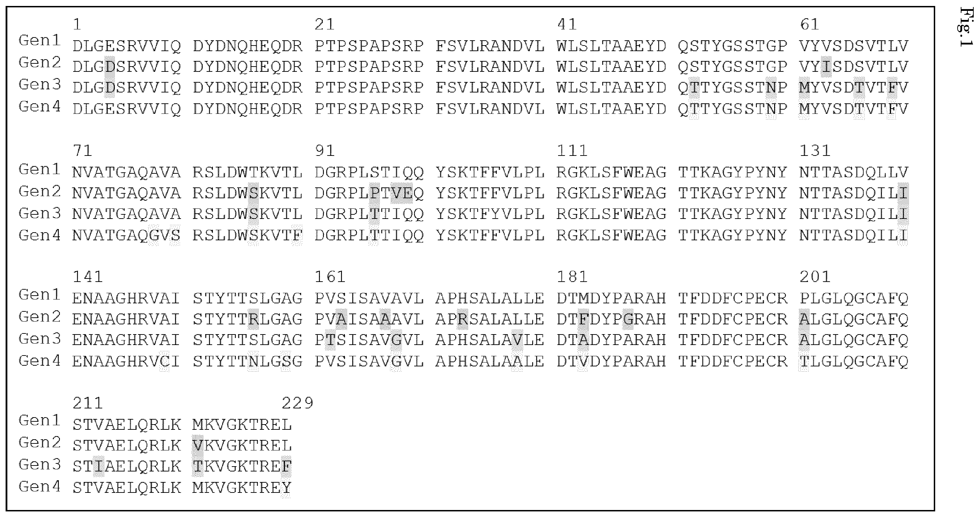

Fig. 1 shows the amino acid sequences set forth in SEQ ID Nos: 1 to 4 as Gen1 to Gen4. Additionally, the identity among the amino acid sequences at positions 21 to 186 within each amino acid sequence is shown in Table 1 below. As demonstrated in Fig. 1 and Table 1, the amino acid identity between the Genotypes 1 to 4 is high.

**[Table 1]**

| Identity (21-186) | | |
|---|---|---|
| Gen1 vs Gen2 | 155/166 | 93.40% |
| Gen1 vs Gen3 | 154/166 | 92.80% |
| Gen1 vs Gen4 | 149/166 | 89.80% |
| Gen2 vs Gen3 | 149/166 | 89.80% |
| Gen2 vs Gen4 | 145/166 | 87.30% |
| Gen3 vs Gen4 | 157/166 | 94.60% |

Amino acids 28 to 175 in the amino acid sequences set forth in SEQ ID NOs: 1 to 4 typically correspond to amino acids 459 to 606 (E2s domain) in the amino acid sequence encoded by the ORF2 of the HEV gene. As the amino acid sequence that needs to be included in the antigen polypeptide (a), it is preferable to be 21 to 175, more preferable to be 21 to 186, and further preferable to be 14 to 186, in the amino acid sequences set forth in SEQ ID NOs: 1 to 4 independently. Described herein is a dimer (heterodimer) below, which is a combination of any one type of polypeptide selected from the group consisting of (3a) to (3c) and any one type of polypeptide (antigen polypeptide (4)) selected from the group consisting of (4a) to (4c), from the viewpoint of making it easier to further purify the heterodimer, as the amino acid sequence that needs to be included in one of the polypeptides (preferably antigen polypeptide (4)), it is preferable to be 14 to 175 in the amino acid sequences set forth in SEQ ID NOs: 3 and 4 (preferably SEQ ID NO: 4), and more preferable to be 14 to 186.

The length of the antigen polypeptide (a) according to the present invention needs to be 200 residues or less, in full length, from any of the amino acid sequences set forth in SEQ ID NOs: 1 to 4. This allows the detection of anti-HEV antibodies with a higher sensitivity than conventional methods. The length of the aforementioned antigen polypeptide (a) is preferably 173 residues or less, and more preferably 166 residues or less.

Additionally, in the natural world, it is possible for the amino acid sequence encoded by a nucleotide sequence to mutate due to changes in the nucleotide sequence. Moreover, with the current level of technology, those skilled in the art can, for example, modify the sequence using known site-directed mutagenesis methods when they obtain information on the amino acid sequence of the antigen polypeptide (a) or information on the nucleotide sequence encoding the same, thus preparing a variant of the antigen polypeptide (a).

Therefore, the antigen polypeptide includes (b) a polypeptide consisting of an amino acid sequence in which one or more amino acids are substituted, deleted, inserted, and/or added in the amino acid sequence which contains amino acids located at position 28 to 175 in the amino acid sequence set forth in any one of SEQ ID NOs: 1 to 4 and which has a full length of 200 residues or less that are contained in the amino acid sequence set forth in that sequence number, namely, at least one polypeptide selected from the group consisting of
(1b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(2b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(3b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3; and
(4b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4 (hereinafter occasionally referred to as "antigen polypeptide (b)").

Here, in amino acid sequences, the term "amino acid sequence in which amino acids have been substituted, deleted, inserted, and/or added" refers to an amino acid sequence in which the amino acid residues within that amino acid sequence have been substituted, deleted, inserted, or added, or an amino acid sequence subjected to a combination of two or more of these alterations. Additionally, "more" signifies an integer of 20, 19, 18, 17, 16, 15, 14, 13, 12, 11, 10, 9, 8, 7, 6, 5, 4, 3, or 2. One or more amino acid residues preferably means 1 or more and 10 or less amino acid residues, more preferably 1 or more and 5 or less, further preferably 1 or more and 3 or less, and most preferably 2 or less.

Moreover, with the current level of technology, those skilled in the art can, for example, upon obtaining information on the amino acid sequence of the aforementioned antigen polypeptide (a), acquire homologous genes from other viruses and the like, based on the nucleotide sequence encoding the same, using techniques such as hybridization (Southern, E. M., J. Mol. Biol., 98, 1975, p. 503-517) or polymerase chain reaction (PCR) (Saiki, R. K. et al., Science, 230, 1985, p. 1350-1354, Saiki, R. K et al., Science, 239, 1988, p. 487-491).

For example, the polypeptide encoded by the homologous gene obtained by such a method usually has high homology or identity with the amino acid sequence of the antigen polypeptide (a). The antigen polypeptide includes (c) a polypeptide composed of an amino acid sequence having 80% or more homology (preferably identity) with the amino acid sequence which contains amino acids located at position 28 to 175 in the amino acid sequence set forth in any one of SEQ ID NOs: 1 to 4 and which has a full length of 200 residues or less that are contained in the amino acid sequence set forth in that sequence number, namely, at least one polypeptide selected from the group consisting of
(1c) a polypeptide consisting of an amino acid sequence having 80% or more homology (preferably identity) with the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(2c) a polypeptide consisting of an amino acid sequence having 80% or more homology (preferably identity) with the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(3c) a polypeptide consisting of an amino acid sequence having 80% or more homology (preferably identity) with the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3; and
(4c) a polypeptide consisting of an amino acid sequence having 80% or more homology (preferably identity) with the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4
(hereinafter occasionally referred to as "antigen polypeptide (c)").

When referring to the homology of amino acid sequences, when aligning the target amino acid sequence with the subject amino acid sequence using software for amino acid sequence analysis, the target amino acids that align with the subject amino acids on the subject amino acid sequence can be either the same amino acids as the subject amino acids or amino acids with the same properties as the subject amino acids. When referring to the identity of amino acid sequences, the target amino acids that align with the subject amino acids on the subject amino acid sequence are the same amino acids as the subject amino acids. Groups of amino acids with the same properties are well known in the technical field to which the present invention belongs, and can be, for example, categorized as acidic amino acids (aspartic acid and glutamic acid); basic amino acids (lysine, arginine, and histidine); and among neutral amino acids, amino acids with hydrocarbon chains (glycine, alanine, valine, leucine, isoleucine, and proline), amino acids with hydroxyl groups (serine and threonine), sulfur-containing amino acids (cysteine and methionine), amino acids with amide groups (asparagine and glutamine), amino acids with imino groups (proline), and amino acids with aromatic groups (phenylalanine, tyrosine, and tryptophan).

Such homology and identity of amino acid sequences can be determined, for example, using the BLASTP program (Altschul et al., J. Mol. Biol., 215, 1990, p. 403-410). Furthermore, it suffices that the homology with the amino acid sequence of the aforementioned antigen polypeptide (a) is 80% or more, but preferably, it is 85% or more, 90% or more, or 95% or more (for example, 96% or more, 97% or more, 98% or more, or 99% or more). More preferably, in terms of identity, it suffices that it is 80% or more, but preferably, it is 85% or more, 90% or more, or 95% or more (for example, 96% or more, 97% or more, 98% or more, or 99% or more).

In the present invention, a dimer consisting of any one polypeptide selected from the group consisting of the claimed (3a) to (3c), and any one polypeptide selected from the group consisting of the claimed (1a) to (1c), (2a) to (2c), and (4a) to (4c) is used from the viewpoints of being able to detect more samples in which anti-HEV antibodies exist (for example, being able to detect more positive samples) and having higher detection sensitivity (for example, being able to detect even small anti-HEV antibody levels). While it is known that HEV has genotypes 1 to 4 as described above, conventional kits for detecting hepatitis E virus infection use antigen polypeptides derived from genotypes 1 and 4. On the other hand, HEV detected in Japan to date are of genotypes 1, 3, and 4. In Hokkaido, although genotype 4 is sporadically detected, the predominant genotype is 3. Therefore, it is thought that the conventional kits produced false negatives in a considerable number of samples. Considering the above, the present inventors infer that by using the aforementioned antigen polypeptide (3) derived from genotype 3 of HEV as the HEV antigen, it is possible to reduce the frequency of such detection failures.

In the present invention, any one polypeptide selected from the group consisting of (3a) to (3c) and any one polypeptide selected from the group consisting of (1a) to (1c), (2a) to (2c), and (4a) to (4c) forms a dimer (heterodimer), more preferably such that any one polypeptide selected from the group consisting of (3a) to (3c) and any one polypeptide selected from the group consisting of (1a) to (1c) and (4a) to (4c) forms a dimer (heterodimer), and further preferably such that any one polypeptide selected from the group consisting of (3a) to (3c) and any one polypeptide selected from the group consisting of (1a) to (1c) forms a dimer (heterodimer). As described later, the present inventors have found that the antigen polypeptide according to the present invention is easily purified as a heterodimer, and in the heterodimer, a synergistic effect of the combination of the two types of polypeptides is higher, that is, it is possible to detect anti-HEV antibodies in samples where either of these polypeptides alone cannot detect anti-HEV antibodies, or where the sensitivity becomes lower, and furthermore, its detection sensitivity becomes even higher.

### [Method for Producing Antigen Polypeptide]

The antigen polypeptide according to the present invention can be obtained by appropriately using a known method or a method similar thereto. For example, it can be obtained by a production method that includes cultivating a host cell into which at least one selected from the group consisting of a nucleotide encoding the aforementioned antigen polypeptide and a vector containing the nucleotide has been introduced, and collecting the polypeptide expressed in the host cell. More specifically, for example, first, RNA encoding the aforementioned antigen polypeptide from HEV is obtained using conventional methods, and cDNA is synthesized. The cDNA may be synthetic DNA that has been chemically synthesized artificially based on the amino acid sequence of the antigen polypeptide according to the present invention. Subsequently, from such cDNA, a DNA fragment encoding the aforementioned antigen polypeptide or an expression vector containing the same is prepared, and by cultivating the transformant introduced into the host cell, the antigen polypeptide according to the present invention is expressed and can be obtained from the culture as a recombinant polypeptide.

The method for obtaining a nucleotide encoding the antigen polypeptide according to the present invention from HEV can include, for example, preparing a cDNA library by connecting cDNA, synthesized based on RNA extracted from HEV, to vectors such as plasmid vectors, phage vectors, cosmid vectors, BAC vectors, and PAC vectors, and using a primer prepared based on the nucleotide sequence encoding the aforementioned antigen polypeptide to amplify DNA fragments containing the target nucleotide sequence, and connecting these fragments to a suitable vector, if necessary.

The expression vector is a vector that can be replicated within host cells, and also contains the polypeptide in a state that allows for the expression of the polypeptide encoded by its nucleotide sequence within the host cells. Such an expression vector can be constructed based on an autonomous replication vector, which exists as an independent entity separate from chromosomes, and its replication does not depend on the replication of chromosomes, for example, a plasmid (such as pBR322). Additionally, the expression vector may be constructed based on phage DNA (such as λ phage) that, on introduction into a host cell, is incorporated into the genome of the host cell, and is replicated along with the chromosome it has been incorporated into.

The procedure and method for constructing the expression vector can be appropriately employed from known methods or methods similar thereto. For example, to insert a DNA fragment containing the target nucleotide sequence into the expression vector, methods are employed such as cleaving the DNA fragment with an appropriate restriction enzyme and inserting it into a restriction enzyme site or multi-cloning site of an appropriate plasmid to link it to the plasmid. The expression vector, to actually express the antigen polypeptide by introducing it into the host cell, preferably includes, in addition to the nucleotide sequence encoding the antigen polypeptide, a nucleotide sequence controlling its expression (such as a sequence encoding a promoter or terminator), a nucleotide sequence inducing expression other than the nucleotide sequence controlling the expression (such as lactose operon), and a gene marker sequence for selecting cells (such as a sequence encoding a drug resistance gene).

The host cells are not particularly limited, but preferably microorganisms, such as filamentous fungi, yeast, E. coli, actinomycetes, and lactic acid bacteria. As the host cells, those that have already been transformed to delete specific functions or mutants may be used as necessary. The method of introducing the DNA fragment or expression vector into these host cells can appropriately employ known methods or methods similar thereto, such as the heat shock method, electroporation method, spheroplast method, or lithium acetate method.

The antigen polypeptide can be collected from the culture product (such as cultured microbial cells) by culturing the transformant, formed by introducing the DNA fragment or expression vector into the host cell, in an appropriate medium. The culture conditions for the transformant can, for example, be based on the culture conditions for host cell, and those skilled in the art can adjust and set parameters such as temperature, the presence or absence of air addition, oxygen concentration, carbon dioxide concentration, pH of the medium, culture temperature, culture time, and humidity, according to the type of the host cell and the medium used.

The method for collecting the antigen polypeptide from the culture can employ any known method or a method similar thereto as appropriate. For example, a method may be employed in which the antigen polypeptide is expressed within host cells (such as E. coli), and after the culture of the transformant ends, the cultured cells is disrupted, and the insoluble fraction obtained through centrifugation or filtration is used as a crude purified product. This insoluble fraction can further be washed as needed and, for example, after solubilizing with denaturants such as urea or guanidine hydrochloride, it can be purified using salt precipitation, dialysis, organic solvent precipitation, membrane separation, or chromatographic separation individually or in combination. Alternatively, the antigen polypeptide, to which a purification tag (such as HIS tag or trpE tag) has been added, can be expressed within host cells (such as E. coli), and the crude extract can then be purified by passing it through a column for purifying the tagged protein (such as a Ni column or an affinity column) and then eluting the tagged protein. Any of these purification methods can be used individually or in combination.

The antigen polypeptide according to the present invention, for example, can be easily purified using a Ni column by expression with an added HIS tag. Additionally, by expression with an added trpE tag, the expression efficiency can be improved, or it can be purified using an affinity column with an anti-trpE antibody. As an example of the purification method for the antigen polypeptide according to the present invention, more specifically, the crude purified product obtained by expression with an added HIS tag is first washed appropriately as necessary and then solubilized. The solution used for this solubilization is preferably a solution containing 4 to 8M urea, for example. Then, it is dialyzed in PBS (pH 7.3), and after dialysis, the supernatant of the centrifuge is captured in a Ni column, washed as necessary, and eluted with imidazole or the like. This allows obtaining the purified product of the antigen polypeptide as an eluate. The eluate can be dialyzed again as needed.

As the antigen polypeptide according to the present invention is the aforementioned dimer (heterodimer), an embodiment of the production method thereof is as follows: first, for example, a HIS tag is added to one of the polypeptides (Polypeptide A) that constitute the desired heterodimer and it is expressed, and the other polypeptide (Polypeptide B) is expressed without adding a HIS tag, and each is then solubilized using the method described above. The solution used for this solubilization is preferably a solution containing 4 to 8M urea, for example. Next, these are mixed and then dialyzed. The solution used for this dialysis is preferably PBS (pH 7.3), for example. As a result, although most monomers and homodimers of polypeptide A with a HIS tag can be recovered as a precipitate, by forming a heterodimer with polypeptide B, which does not have a HIS tag, it can be solubilized and recovered as a supernatant. This is hypothesized by the present inventors to be due to the chaperone effect of polypeptide B on polypeptide A. Next, by introducing the aforementioned supernatant into a Ni column, the monomer and homodimer of polypeptide B without a HIS tag can be removed as a pass-through fraction, so that by eluting the proteins that has been captured in the Ni column, it is possible to obtain a heterodimer of polypeptide A and polypeptide B. The solution used for this elution can be an imidazole solution, for example.

### [Detection Step]

In the present invention, the detection method for anti-HEV antibodies using the aforementioned antigen polypeptide is not particularly limited, as long as the method can detect anti-HEV antibodies, and conventionally known or equivalent methods, or a combination thereof, can be appropriately employed. Examples thereof include, but are not particularly limited to, immunological techniques such as the sandwich method, competitive method, and immunoturbidimetry, as well as detection methods based on these principles. Such detection methods can generally employ, for example, methods using microplates or particles as carriers, such as ELISA, digital ELISA, CLEIA (Chemiluminescent Enzyme Immunoassay), CLIA (Chemiluminescent Immunoassay), ECLIA (Electrochemiluminescence Immunoassay), and RIA (Radioimmunoassay); immunochromatography; surface plasmon resonance analysis; and detection methods based on fluorescence resonance energy transfer.

From the viewpoint of building a measurement system with higher sensitivity and specificity, the sandwich method is preferable as a detection method in the present invention. Hereinafter, a more specific embodiment of the detection step according to the present invention is described using the sandwich method as an example.

In the case of using the sandwich method, an embodiment of the sandwich method according to the present invention is such that
the detection step comprises a step of bringing the sample into contact with a capture body and a labeled body, and
the capture body comprises a water insoluble carrier and a probe molecule capable of binding to the anti-HEV antibody, and the labeled body comprises a labeling substance and the antigen polypeptide (at least one of the polypeptides) (hereinafter occasionally referred to as the "first embodiment"); and
detection step comprises a step of bringing the sample into contact with a capture body and a labeled body, wherein
the capture body comprises a water insoluble carrier and the antigen polypeptide, and
the labeled body comprises a labeling substance and a probe molecule capable of binding to the anti-HEV antibody (hereinafter occasionally referred to as the "second embodiment"). From the viewpoint of reducing nonspecific reactions, the first embodiment is preferable.

In the sandwich method, a probe molecule capable of binding to the anti-HEV antibody (hereinafter occasionally simply referred to as the "probe molecule") and at least one of the aforementioned polypeptides (hereinafter, the "antigen polypeptide") may be provided to either the capture body or the labeled body, but one is included in the capture body, and the other is included in the labeled body. This allows the anti-HEV antibody in the sample to be captured by the capture body and labeled by the labeled body. Therefore, by detecting a signal derived from the labeling substance, the anti-HEV antibody can be detected with high accuracy and ease.

The detected signal can be semi-quantified or quantified as needed to determine the anti-HEV antibody level. The term "signal" includes coloration (color development), extinction, reflected light, light emission, fluorescence, and radiation from a radioactive isotope, depending on the labeling substance. In addition to what can be confirmed by the naked eye, it also includes what can be confirmed by a detection method or device according to the type of signal. In the present invention, the anti-HEV antibody level can be measured, for example, using a calibration curve with a standard sample, but from a more convenient and faster viewpoint, the signal intensity may be treated directly as the anti-HEV antibody level in the present invention.

Such sandwich methods include the two-step forward sandwich method (a method that sequentially performs a reaction between the capture body and the anti-HEV antibody in the sample, and a reaction between the anti-HEV antibody bound to the capture body and the labeled body), the reverse sandwich method (a method that pre-reacts the labeled body and the anti-HEV antibody in the sample, and reacts the resulting complex with the capture body), and the one-step method (a method that performs the reaction between the anti-HEV antibody in the sample, the capture body, and the labeled body in one simultaneous step), any of which can be employed.

For example, in the forward sandwich method in the first or second embodiment, first, the sample and the capture body are brought into contact, and the anti-HEV antibody is captured on the capture body through the binding of the probe molecule (or antigen polypeptide) of the capture body and the anti-HEV antibody (first reaction: capturing step). Then, the labeled body is brought into contact with the anti-HEV antibody captured on the capture body, and is labeled through the binding of the antigen polypeptide (or probe molecule) of the labeled body and the anti-HEV antibody (second reaction: labeling step). Through this reaction, a complex containing capture body - anti-HEV antibody - labeled body is formed. After removing unbound samples and labeled bodies as needed by washing (washing step), the signal derived from the labeling substance is detected by a predetermined method according to the labeling substance (detection step).

### [Water Insoluble Carrier]

The water insoluble carrier primarily supports the probe molecules or the antigen polypeptide, functioning as an immobilization carrier and is a water insoluble substance. In the present invention, a "water insoluble substance" refers to a substance that is insoluble in water under normal temperature and pressure conditions (solubility in water is 0.001 g/mL or less, preferably 0.0001 g/mL or less, hereinafter the same).

As for the material of such a water insoluble carrier, those commonly used in immunological measurements and similar measurements can be used without particular restriction. For example, these can be at least one selected from the group consisting of polymers (such as polystyrene, (meth)acrylic acid ester, polymethyl methacrylate, polyimide, and nylon), gelatin, glass, latex, silica, metals (such as gold and platinum), and metal compounds (such as iron oxide, cobalt oxide, and nickel ferrite). Additionally, as the material for the water insoluble carrier, composite materials thereof as well as composite materials of these substances and other substances can be used. For example, organic-inorganic composite materials composed of at least one organic polymer from the group consisting of the above-mentioned polymers, gelatin, and latex, and at least one metal compound from the group consisting of iron oxide (such as spinel ferrite), cobalt oxide, and nickel ferrite (such as magnetic particles) can be used. Furthermore, the water insoluble carrier may be surface-modified with active groups such as carboxy groups, epoxy groups, tosyl groups, amino groups, hydroxyl groups, isothiocyanate groups, isocyanate groups, azide groups, aldehyde groups, carbonate groups, allyl groups, aminooxy groups, maleimide groups, and thiol groups.

In the present invention, there is no particular restrictions on the shape of the water insoluble carrier, and it can be in any form such as a plate, fiber, film, or particle. As such water insoluble carriers, conventionally known ones can be used, and commercially available ones can also be used as appropriate.

### [Probe Molecules Capable of Binding to Anti-HEV Antibodies]

As for the probe molecules capable of binding to anti-HEV antibodies, examples include antigens; antibodies; binding proteins such as Protein A, Protein G, and Protein L; avidins such as Avidin D and streptavidin; lectins; galectins, glycans receptors, immune receptors, and the like. The term "antibodies" encompasses not only complete antibodies but also antibody fragments (such as Fab, Fab', F(ab')₂, Fv, single-chain antibodies, and diabodies) and low-molecular-weight antibodies conjugated with the variable region of an antibody. Among these, the probe molecule is preferably an antibody capable of binding to anti-HEV antibodies. Such antibodies, for example, can be selected from anti-IgG antibodies, anti-IgM antibodies, anti-IgA antibodies, anti-IgE antibodies, and anti-IgD antibodies according to the isotype of the target anti-HEV antibody to be detected, and two or more of these may be combined. These isotype-specific antibodies can be suitably prepared using conventionally known methods, or commercially available ones can be used as appropriate.

### [Labeling Substances]

Any labeling substances that are used as labeling substances in known immunological measurement methods or similar methods can be used without any particular limitation. Examples thereof include enzymes; luminescent substances such as acridinium derivatives; fluorescent substances such as europium; fluorescent proteins such as allophycocyanin (APC) and phycoerythrin (R-PE); radioactive substances such as ¹²⁵I; low-molecular-weight labeling substances such as fluorescein isothiocyanate (FITC) and rhodamine isothiocyanate (RITC); gold particles; avidins; biotin; latex; dinitrophenyl (DNP); and digoxigenin (DIG). These can be used individually or in any combination of two or more. For example, if an enzyme is used as the labeling substance, by adding a chromogenic substrate, fluorescent substrate, chemiluminescent substrate, or the like as a substrate, various signals can be detected according to the substrate. Examples of the enzyme include, but are not limited to, peroxidases (such as POD: horseradish peroxidase (HRP)), alkaline phosphatase (ALP), β-galactosidase (β-gal), glucose oxidase, or luciferase.

### [Capture Body]

The labeled body is a complex that includes the water insoluble carrier and the probe molecule or the antigen polypeptide, and is formed by the direct or indirect binding of the water insoluble carrier with the probe molecule or the antigen polypeptide.

In the capture body, the content of the probe molecule or the antigen polypeptide is not particularly limited and can be adjusted as appropriate according to, for example, the ease of binding these with the anti-HEV antibody. For example, if the water insoluble carrier is a plate, the mass per surface area of 1 cm² (or the total in the case of a combination of two or more types) is preferably 0.01 to 1.5 µg.

The capture body can be produced by binding the probe molecule or the antigen polypeptide to the water insoluble carrier. Such a production method may employ an appropriate conventionally known method or a method similar thereto. The water insoluble carrier may be directly or indirectly bound with the probe molecule or the antigen polypeptide (hereinafter occasionally referred to as the "supported body").

In the case of direct binding, for example, the water insoluble carrier and/or the supported body can be ones having active groups such as carboxy groups, epoxy groups, tosyl groups, amino groups, hydroxyl groups, isothiocyanate groups, isocyanate groups, azide groups, aldehyde groups, carbonate groups, allyl groups, aminooxy groups, maleimide groups, thiol groups, and pyridyl disulfide groups. Alternatively, those active groups may be added as needed. Binding them makes it possible to directly fix the supported body to the water insoluble carrier. Also, when the water insoluble carrier is a plate, the plate can be coated with the supported body, which can be blocked with sodium caseinate, bovine serum albumin (BSA), or the like as necessary, followed by drying to fix the supported body directly to the water insoluble carrier.

In the case of indirect binding, for example, the supported body can be indirectly fixed to the water insoluble carrier by binding, for example, through a linker that binds to the supported body, such as an oligopeptide containing polyhistidine, polyethylene glycol, cysteine, and/or lysine. The linker is not particularly limited, and examples thereof include secondary antibodies that can bind to the supported body (preferably the probe molecule), Protein G, Protein A, photo-cleavable linkers, and linker molecules with the above active groups (such as hydrazine salts and hydrazides). Also, some modification may be performed on the supported body (preferably the probe molecule), and a substance that captures the modified part can be fixed on the water insoluble carrier, which allows the supported body to be fixed on the water insoluble carrier. For example, biotin can be mentioned as a typical example of the modified part, and streptavidin can be mentioned as a typical example of a substance that captures the modified part, but their examples are not limited to these.

The ratio of the water insoluble carrier and the supported body used in these reactions can be appropriately selected to achieve the preferable range in the above-mentioned capture body. If necessary, to prevent nonspecific adsorption to the supported body or the water insoluble carrier, a suitable blocking agent (such as bovine serum albumin or gelatin) may be used to block the water insoluble carrier. Furthermore, when such a capture body is used, and if the supported body is the probe molecule, commercially available products can be appropriately used.

### [Labeled Body]

The labeled body is a complex that includes the labeling substance and the probe molecule or the antigen polypeptide, and is formed by the direct or indirect binding of the labeling substance with the probe molecule or the antigen polypeptide. In the labeled body, there is no particular restrictions on the content of the labeling substance, the probe molecule, or the antigen polypeptide, and it can be adjusted appropriately depending on the type of the labeling substance.

The labeled body can be produced by binding the labeling substance to the probe molecule or the antigen polypeptide. Such a production method may employ an appropriate conventionally known method or a method similar thereto. The labeling substance may be directly or indirectly bound with the probe molecule or the antigen polypeptide. As for these binding methods, methods similar to the binding methods described in the capture body can be used.

### (Capturing Step)

In the aforementioned capturing step, the method of contact between the anti-HEV antibody and the capture body is not particularly limited, and a conventionally known method or a method similar thereto can be employed as appropriate. For example, if the water insoluble carrier is a plate, one may employ a method of injecting the sample onto it (a supported body-immobilized plate). If the water insoluble carrier is a particle, one may employ a method of adding the capture body (a supported body-immobilized particles) into the sample.

In the reaction between the sample and the capture body during the capturing step, the amount of the sample is not particularly limited and can be adjusted appropriately according to the type, concentration, and the like of the sample. In addition, the conditions of the capturing step are not particularly restricted either and can be adjusted appropriately. For example, it can be carried out at room temperature to 45°C, preferably 20 to 37°C, at a pH of about 6.0 to 9.0, preferably pH of 7.0 to 8.0, for approximately 30 to 90 minutes, preferably around 60 minutes. However, these conditions are not exhaustive.

### (Labeling Step)

In the reaction between the labeled body and anti-HEV antibodies in the aforementioned labeling step, the content (final concentration) of the labeled body in the reaction solution containing the labeled body and anti-HEV antibodies (or the total in the case of a combination of two or more types of labeled bodies) is not particularly limited, as it is appropriately adjusted according to the type, concentration, and the like of the sample. However, from the viewpoint that its excessive use may result in a high background signal, it is preferably 0.01 to 10 µg/mL, more preferably 0.1 to 5 µg/mL, and further preferably 0.2 to 1 µg/mL, for example.

In addition, the conditions of the labeling step are not particularly restricted either and can be adjusted appropriately. For example, it can be carried out at room temperature to 37°C, preferably 20 to 37°C, at a pH of 5.0 to 7.0, preferably pH of 5.5 to 6.5, for approximately 30 to 90 minutes, preferably around 60 minutes. However, these conditions are not exhaustive.

### (Washing Step)

The sandwich method mentioned earlier preferably further includes, after the capturing step and/or labeling step, a washing step of separating the anti-HEV antibodies bound to the capture body from other impurities that are not bound to (that is, not captured by) the capture body to remove these impurities. There are no particular restrictions on the method for removing these impurities, and any conventionally known method or a method similar thereto can be used as appropriate. For example, if the capture body is a supported body-immobilized plate, one may employ a method of removing the liquid phase (supernatant) from the plate, or if the capture body is a supported body-immobilized particle, one may employ a method of recovering these particles through centrifugation or magnetic collection to remove the liquid phase (supernatant). During the washing step, if necessary, the injection and removal of the washing liquid can be repeated. Examples of the washing liquid include known neutral buffer solutions (preferably, pH 6.0 to 9.0) (such as a sodium phosphate buffer, MES, Tris, CFB, MOPS, PIPES, HEPES, tricine buffer, bicine buffer, and glycine buffer). Also, they may be added with stabilizing proteins such as BSA or surfactants.

In the case where the sandwich method is used for the detection step according to the present invention, the sample may be diluted with a diluent as mentioned above. However, if the capture body is the above-described supported body-immobilized particles or the like, the it may be suspended in a suspension medium of those particles (particle solution). Furthermore, other reaction buffers may be added as needed to the reaction system containing the sample, the capture body, and/or the labeled body. There are no particular restrictions on the particle suspension medium or the reaction buffers, but each independently, known buffer solutions (such as a sodium phosphate buffer, MES, Tris, CFB, MOPS, PIPES, HEPES, tricine buffer, bicine buffer, and glycine buffer) can be used. Also, they may each independently be added with stabilizing proteins such as BSA or serum.

### (Detection Step)

In the detection step, a signal is detected according to the labeling substance. For example, if the labeling substance is an enzyme, a signal (such as color development or luminescence) produced by reacting the enzyme with corresponding chromogenic or luminescent substrates is detected. This allows for the detection of the presence or absence of anti-HEV antibodies in the sample based on the presence or absence of the signal. Furthermore, if anti-HEV antibodies are present, the anti-HEV antibody level in the sample can be obtained as the signal quantity. Moreover, if necessary, the anti-HEV antibody level in the sample can be quantified by comparing with the signal quantity in the standard sample.

### <Method for Testing Hepatitis E Virus>

According to the method for detecting hepatitis E virus infection of the present invention, the presence or absence of the hepatitis E virus infection in the sample can be determined, preferably, the presence or absence of hepatitis E virus infection in the subject from whom the sample originates can be detected, by using the presence or absence of anti-HEV antibodies detected by the aforementioned detection step as an indicator. Additionally, information on the degree of hepatitis E virus infection (such as the time of infection and the severity of hepatitis E) in the sample, preferably, in the subject from whom the sample originates can be obtained by using the anti-HEV antibody level detected by the aforementioned detection step as an indicator. Therefore, the present invention also provides a method for testing hepatitis E virus, which includes a step of detecting anti-hepatitis E virus antibodies in a sample derived from a subject using the method for detecting hepatitis E virus infection of the present invention (hereinafter occasionally simply referred to as "test method") .

Specifically, for example, the following embodiments can be mentioned as embodiments of the test method of the present invention:
a method that comprises a detection step of detecting anti-HEV antibodies in a sample derived from a subject with the antigen polypeptide, and a determination step of determining whether or not the subject is infected with HEV using the detected anti-HEV antibodies as an indicator;
a method (a screening method) that comprises a detection step of detecting anti-HEV antibodies in a sample derived from a subject with the antigen polypeptide, and a selection step of selecting subjects who are predicted to be highly likely to have hepatitis E or to develop hepatitis E using the detected anti-HEV antibodies as an indicator; and
a method that comprises a detection step of detecting anti-HEV antibodies in a sample derived from a subject with the antigen polypeptide, and a discrimination step of discriminating the presence or absence of hepatitis E infection in the subject using the detected anti-HEV antibodies as an indicator.

In each of the test methods, in the determination step, selection step, and discrimination step, if anti-HEV antibodies are detected even slightly in the sample in the detection step, it may be acceptable to regard the subject from whom the sample originates as being infected with the hepatitis E virus, having or highly likely to develop hepatitis E, and suffering from hepatitis E (that is, being positive). However, it is preferable to determine, select, or discriminate according to the anti-HEV antibody level detected. For example, comparing the anti-HEV antibody level detected in the detection step with a predetermined cutoff value, it may be acceptable to regard subjects whose anti-HEV antibody level is higher than the cutoff value as being infected with the hepatitis E virus, having or highly likely to develop hepatitis E, or suffering from hepatitis E.

The "cutoff value" is a predetermined value that serves as a criterion for determination based on the anti-HEV antibody level, indicating a boundary value for determination between positive and negative groups. Such a cutoff value is set as appropriate according to the purpose of the test method of the present invention, the detection method for anti-HEV antibodies, the nature of the subject and the sample, dilution conditions, and the like, and thus is not particularly limited. For example, by setting the cutoff value to a relatively low value, the detection sensitivity can be increased, that is, subjects suspected of being positive can be collected to some extent broadly, and on the other hand, by setting the cutoff value to a relatively high value, it is possible to perform each of the test methods with higher accuracy.

According to the test method of the present invention, it becomes possible to provide information for determining a treatment policy specific to hepatitis E, distinguishing it from other types of hepatitis. Furthermore, by identifying subjects who are highly likely to contract hepatitis E (including reactivation), early treatment intervention becomes possible. Moreover, an embodiment of the test method of the present invention includes a method that includes a detection step of detecting anti-HEV antibodies in a sample derived from the subject using the antigen polypeptide, and a prediction step of predicting the timing of HEV infection in the subject using the detected anti-HEV antibodies as an indicator. For example, by appropriately selecting anti-IgG antibodies, anti-IgM antibodies, and anti-IgA antibodies as probe molecules, it is also possible to predict the timing of HEV infection in the subject from the isotype of the anti-HEV antibodies. Note that the test method of the present invention is also a method to assist physicians and the like in diagnosing hepatitis E, or a method to provide information for physicians and the like to diagnose hepatitis E.

### <Antigen Polypeptide and Kit for Detecting Hepatitis E Virus Infection>

The antigen polypeptide for detecting hepatitis E virus infection of the present invention is an antigen polypeptide according to the method for detecting hepatitis E virus infection according to the present invention, and is a dimer consisting of any one polypeptide selected from the group consisting of the claimed (3a) to (3c), and any one polypeptide selected from the group consisting of the above (1a) to (1c), (2a) to (2c), and (4a) to (4c). The antigen polypeptide of the present invention is as described above, including its preferable embodiments. In addition, the kit for detecting hepatitis E virus infection of the present invention is a kit according to the method for detecting hepatitis E virus infection according to the present invention, and includes the antigen polypeptide of the present invention.

The antigen polypeptide included in the kit of the present invention may be composed only of the aforementioned antigen polypeptide or it may be a composition containing the same. The composition may further contain additional ingredients, and examples of these additional ingredients include, but are not particularly limited to, sterilized water, physiological saline, surfactants, and preservatives. These can be used individually or in any combination of two or more.

The kit of the present invention preferably further comprises a capture body including the water insoluble carrier and the probe molecule. In this case, the antigen polypeptide is preferably the labeled body labeled with the labeling substance. Also, the kit of the present invention preferably further comprises the labeled body furnished with the labeling substance and the probe molecule. In this case, the antigen polypeptide is preferably the capture body supported by the water insoluble carrier. The capture body and the labeled body, comprising preferable embodiments thereof, are as previously stated. Furthermore, in this case, the capture body and the labeled body can each independently be in solid (powder) form or in a liquid form that is dissolved or dispersed in a buffer solution (for example, particle dispersion for the capture body).

In the case of liquid form, the concentration of the capture body and the labeled body in each solution is not particularly limited, but is each independently preferably 0.01 to 10 µg/mL, more preferably 0.1 to 5.0 µg/mL, and further preferably 0.2 to 1.0 µg/mL, for example.

The kit of the present invention may further comprise configurations for typical immunological measurement methods such as ELISA, CLEIA, and immunochromatography, as well as methods similar thereto. For example, if the aforementioned sandwich method is used as the principle of the detection method according to the present invention, it may also include at least one selected from the group consisting of magnetic beads or plates for immobilizing the supported bodies; the labeling substances; the standard samples (each concentration); control reagents; the particle suspension media; the reaction buffers; and the washing liquids. Also, if the labeling substance is an enzyme, it may also comprise substrates and reaction stoppers necessary for the detection and quantification of the labeling substance.

Furthermore, the kit of the present invention may also comprise a pretreatment solution for pretreating the diluent or the sample as needed; a dilution cartridge; a reaction stopper or neutralizer for pretreatment. If immunochromatography is employed as the sandwich method, it may also comprise a device containing a zone that supports the capture body and/or the labeled body. The device can include other components suitable for immunochromatography, such as a development liquid pad and an absorption pad. Additionally, the kit of the present invention may also include instructions for use of the kit.

### [Example]

Hereinafter, the present invention will be described in more detail based on the Examples and Comparative Examples. Examples not relating to the claimed antigen polypeptide being a dimer consisting of any one polypeptide selected from the group consisting of the claimed (3a) to (3c), and any one polypeptide selected from the group consisting of the claimed (1a) to (1c), (2a) to (2c), and (4a) to (4c) are for comparison reasons only.

### (Preparation Example 1) Cloning of the E2s gene of HEV genotype 3 (HEVG3E2s)

### (1-1) Cloning of HEV genotype 3 gene by RT-PCR

For HEV genotype 3 (HEVG3, GenBank Accession No.: AB481229), cloning of the HEV gene was performed by RT-PCR. Specifically, first, RNA (HEV-RNA) was extracted and purified from 100 µL of HEVG3 sample (HEVG3: about 10⁴ copies) using High Pure Viral RNA Kit (manufactured by Roche). Purified HEV-RNA at 11 µL, reverse primer G3AS1 (2 µM, SEQ ID NO: 5) at 1 µL, and dNTP (each 10 mM) at 1 µL were dispensed into a 0.5 mL tube, then heated at 65°C for 5 minutes and quickly cooled on ice. Next, to each tube, RNase inhibitor (manufactured by Takara Shuzo) at 1 µL, 100 mM DTT at 1 µL, 5 × SSIV buffer solution (manufactured by Invitrogen) at 4 µL, and reverse transcriptase SSIV (manufactured by Invitrogen) at 1 µL were added to prepare a reaction solution at 20 µL. The above reaction solution was reacted at 50°C for 10 minutes, and then heated at 80°C for 10 minutes to deactivate the enzymes, and a cDNA synthesis reaction solution containing cDNA was obtained.

The nested-PCR method was utilized as PCR to enhance the amplification sensitivity and specificity of the detection DNA. Specifically, first, a first PCR was performed using two types of primers (1st step PCR), and then a second PCR was performed using two types of primers located inside the DNA sequence of that PCR product (2nd step PCR). For the 1st step PCR, primer G3S1 (SEQ ID NO: 6) and the aforementioned reverse primer G3AS1 were used, while for the 2nd step PCR, primer G3S2 (SEQ ID NO: 7) and primer G3AS2 (SEQ ID NO: 8) were used.

The reaction solution for the 1st step PCR consisted of 5 µL of the aforementioned cDNA synthesis reaction solution, 2 µL each of two types of primers for the 1st step PCR (each 50 pmole), 10 µL of KAPATaq Extra HS RM + dye (manufactured by KAPA Biosystems), and 3 µL of sterilized water, for a total volume of 20 µL. The 1st step PCR reaction was carried out for 30 cycles under the conditions of heating at 95°C for 3 minutes, denaturation: at 95°C for 30 seconds, annealing: at 55°C for 30 seconds, and extension: at 72°C for 2 minutes.

The reaction solution for the 2nd step PCR consisted of 2 µL of the 1st step PCR reaction termination solution, 2 µL each of two types of primers for the 2nd step PCR (each 50 pmole), 9 µL of KAPATaq Extra HS RM + dye, and 7 µL of sterilized water, for a total volume of 20 µL. The reaction was carried out under the same conditions as the 1st step PCR reaction.

The 2nd step PCR reaction termination solution at 10 µL was subjected to agarose gel electrophoresis, and the specifically amplified DNA fragments of approximately 5 kbp in length were recovered from the agarose gel using the QIAquick Gel Extraction Kit (manufactured by Qiagen). These fragments were cloned into pGEM-T Easy (manufactured by Promega) to obtain pGEM-HEV3 containing the nucleotide sequence of ORF2 of the HEV genotype 3 gene.

### (1-2) Cloning of the genotype 3 E2s (G3E2s) gene into the expression vector

With the above pGEM-HEV3 as a template, PCR was performed using the primer G3E2s-his452-S (SEQ ID NO: 9) and the primer G3E2s-617-AS (SEQ ID NO: 10) to amplify a DNA fragment (about 530 bp) encoding G3E2s. The primer was designed to add at the 5'-end of the amplified DNA fragment, in order from its 5'-end, the recognition sequence of the restriction enzyme EcoRI and a HIS tag, and at the 3'-end, in order from its 5'-end, a stop codon and the recognition sequence of the restriction enzyme BamHI. By utilizing these two types of restriction enzymes, the amplified DNA fragment was cloned into the EcoRI-BamHI site of the pAT-trp-trpE expression vector (Escherichia coli trpE protein expression vector, a vector described in Japanese Patent No. 3217600) to obtain the vector, pAT-trp-trpE-his-G3E2s (with HIS tag, trpE present), containing a nucleotide sequence encoding the amino acids of 166 residues in full length (G3E2s) from the 21st to 186th in the amino acid sequence set forth in SEQ ID NO: 3, which is part of ORF2 of the HEV genotype 3 gene.

In addition, PCR was performed using the HIS tag-free primer: primer G3E2s-452-S (SEQ ID NO: 11) and the above-described primer G3E2s-617-AS to amplify a DNA fragment (about 510 bp) encoding G3E2s. The primer was designed to add at the 5'-end of the amplified DNA fragment the recognition sequence of the restriction enzyme EcoRI, and at the 3'-end, in order from its 5'-end, a stop codon and the recognition sequence of the restriction enzyme BamHI. By utilizing these two types of restriction enzymes, the amplified DNA fragment was cloned into the EcoRI-BamHI site of the pAT-trp-trpE expression vector to obtain the vector, pAT-trp-trpE-G3E2s (without HIS tag, trpE present), containing a nucleotide sequence encoding the amino acids of 166 residues in full length (G3E2s) from the 21st to 186th in the amino acid sequence set forth in SEQ ID NO: 3, which is part of ORF2 of the HEV genotype 3 gene.

### (Preparation Example 2) Cloning of the E2s genes of other HEV genotypes

### (2-1) Cloning of the genotype 1 E2s (G1E2s) gene into the expression vector

For HEV genotype 1 (HEVG1), the nucleotide sequence of its ORF2 and the amino acid sequence encoded by it were obtained from the DNA Data Bank of Japan (DDBJ) (DDBJ Accession No.: D11092). The nucleotides encoding amino acids 394 to 617 of the obtained amino acid sequence (ORF2) (G1E2s 394 to 617, the sequence is set forth in SEQ ID NO: 12) were synthesized on request to Eurofins Genomics, and pEX-A-HEV1 containing the nucleotide sequence set forth in SEQ ID NO: 12 was obtained.

With the above pEX-A-HEV1 as a template, PCR was performed using the primer G1E2s-his452-S (SEQ ID NO: 13) and the primer G1E2s-617-AS (SEQ ID NO: 14) to amplify a DNA fragment (about 530 bp) encoding G1E2s. The primer was designed to add at the 5'-end of the amplified DNA fragment, in order from its 5'-end, the recognition sequence of the restriction enzyme EcoRI and a HIS tag, and at the 3'-end, in order from its 5'-end, a stop codon and the recognition sequence of the restriction enzyme BamHI. By utilizing these two types of restriction enzymes, the amplified DNA fragment was cloned into the EcoRI-BamHI site of the pAT-trp-trpE expression vector to obtain the vector, pAT-trp-trpE-his-G1E2s (with HIS tag, trpE present), containing a nucleotide sequence encoding the amino acids of 166 residues in full length (G1E2s) from the 21st to 186th in the amino acid sequence set forth in SEQ ID NO: 1, which is part of ORF2 of the HEV genotype 1 gene.

### (2-2) Cloning of HEV genotype 4 gene by RT-PCR

For HEV genotype 4 (HEVG4, GenBank Accession No.: AB481227), cloning was performed by RT-PCR in the same manner as in (1-1). Specifically, first, RNA (HEV-RNA) was extracted and purified from 100 µL of HEVG4 sample (HEVG4: about 10⁴ copies) using High Pure Viral RNA Kit (manufactured by Roche). Purified HEV-RNA at 11 µL, reverse primer G4AS1 (2 µM, SEQ ID NO: 15) at 1 µL, and dNTP (each 10 mM) at 1 µL were dispensed into a 0.5 mL tube, then heated at 65°C for 5 minutes and quickly cooled on ice. Next, to each tube, RNase inhibitor (manufactured by Takara Shuzo) at 1 µL, 100 mM DTT at 1 µL, 5 × SSIV buffer solution (manufactured by Invitrogen) at 4 µL, and reverse transcriptase SSIV (manufactured by Invitrogen) at 1 µL were added to prepare a reaction solution at 20 µL. The above reaction solution was reacted at 50°C for 10 minutes, and then heated at 80°C for 10 minutes to deactivate the enzymes, and a cDNA synthesis reaction solution containing cDNA was obtained.

For the 1st step PCR, primer G4S1 (SEQ ID NO: 16) and the reverse primer G4AS1 were used, and for the 2nd step PCR, primer G4S2 (SEQ ID NO: 17) and primer G4AS2 (SEQ ID NO: 18) were used.

The reaction solution for the 1st step PCR consisted of 5 µL of the aforementioned cDNA synthesis reaction solution, 2 µL each of two types of primers for the 1st step PCR (each 50 pmole), 10 µL of KAPATaq Extra HS RM + dye (manufactured by KAPA Biosystems), and 3 µL of sterilized water, for a total volume of 20 µL. The 1st step PCR reaction was carried out for 30 cycles under the conditions of heating at 95°C for 3 minutes, denaturation: at 95°C for 30 seconds, annealing: at 55°C for 30 seconds, and extension: at 72°C for 2 minutes.

The reaction solution for the 2nd step PCR consisted of 2 µL of the 1st step PCR reaction termination solution, 2 µL each of two types of primers for the 2nd step PCR (each 50 pmole), 9 µL of KAPATaq Extra HS RM + dye, and 7 µL of sterilized water, for a total volume of 20 µL. The reaction was carried out under the same conditions as the 1st step PCR reaction.

The 2nd step PCR reaction termination solution at 10 µL was subjected to agarose gel electrophoresis, and the specifically amplified DNA fragments of approximately 5 kbp in length were recovered from the agarose gel using the QIAquick Gel Extraction Kit (manufactured by Qiagen). These fragments were cloned into pGEM-T Easy (manufactured by Promega) to obtain pGEM-HEV4 containing the nucleotide sequence of ORF2 of the HEV genotype 4 gene.

### (2-3) Cloning of the genotype 4 E2s (G4E2s) gene into the expression vector

With the above pGEM-HEV4 as a template, PCR was performed using the primer G4E2s-his445-S (SEQ ID NO: 19) and the primer G4E2s-606-AS (SEQ ID NO: 20) to amplify a DNA fragment (about 530 bp) encoding G4E2s. The primer was designed to add at the 5'-end of the amplified DNA fragment, in order from its 5'-end, the recognition sequence of the restriction enzyme EcoRI and a HIS tag, and at the 3'-end, in order from its 5'-end, a stop codon and the recognition sequence of the restriction enzyme BamHI. By utilizing these two types of restriction enzymes, the amplified DNA fragment was cloned into the EcoRI-BamHI site of the pAT-trp-trpE expression vector to obtain the vector, pAT-trp-trpE-his-G4E2s (with HIS tag, trpE present), containing a nucleotide sequence encoding the amino acids of 162 residues in full length (G4E2s) from the 14th to 175th in the amino acid sequence set forth in SEQ ID NO: 4, which is part of ORF2 of the HEV genotype 4 gene.

### (Preparation Example 3) Expression and purification of polypeptide (E2s)

The four types of expression plasmids prepared in Preparation Examples 1 and 2 were used to express and purify four types of polypeptides: trpE-his-G3E2s, trpE-G3E2s, trpE-his-G1E2s, and trpE-his-G4E2s. Specifically, first, Escherichia coli XL1-Blue, having the four types of expression plasmids prepared in Preparation Examples 1 and 2, was inoculated into 40 mL of LB medium containing 50 µg/mL ampicillin, and cultured overnight at 37°C. This 40 mL of culture fluid was transferred into 4 L of M9-CA medium containing 50 µg/mL ampicillin (0.6% Na₂HPO₄, 0.5% KH₂PO₄, 0.5% NaCl, 0.1% NH₄Cl, 0.1 mM CaCl₂, 2 mM MgSO₄, 0.5% casamino acid, and 0.2% glucose) and cultured at 37°C. Indoleacrylic acid was added so that the final concentration was 40 mg/L when OD600 = 0.3, and the solution was further cultured for 16 hours. This culture fluid was centrifuged to collect approximately 12 g of bacterial bodies.

Next, to the obtained bacterial bodies, 60 mL of lysis buffer (50 mM Tris-HCl (pH 8.0), 30 mM NaCl, and 5 mM EDTA) was added and suspended, and 12 mg of lysozyme (manufactured by SEIKAGAKU CORPORATION) was added, followed by reaction at 37°C for 1 hour. After the reaction, the Escherichia coli membrane was broken by ultrasonic disruption for 150 seconds (1), and the supernatant (2) was removed by centrifugation under the conditions of 15000 rpm, 30 minutes, and 4°C, to thereby obtain an insoluble fraction containing a fusion peptide of a polypeptide encoded by each target nucleotide sequence and trpE.

Then, to the aforementioned insoluble fraction, 55 mL of washing buffer containing a surfactant (25 mM Tris-HCl (pH 8.0), 5 mM EDTA, and 0.1% NP-40) was added and suspended, and the supernatant (3) was removed by centrifugation under the conditions of 15000 rpm, 30 minutes, and 4°C, to thereby obtain a washed insoluble fraction. Furthermore, to the aforementioned insoluble fraction, 55 mL of washing buffer (25 mM Tris-HCl (pH 8.0) and 5 mM EDTA) was added and resuspended, and the supernatant (4) was removed by centrifugation under the conditions of 15000 rpm, 30 minutes, and 4°C, to thereby obtain a re-washed insoluble fraction. Then, to the re-washed insoluble fraction, 55 mL of solubilization buffer (4M urea, 25 mM Tris-HCl (pH 8.0), and 5 mM EDTA) was added, to thereby obtain a solubilized matter (5) in which the fusion peptide was solubilized.

Next, the solubilized matter was placed into a dialysis bag (MWCO: 10 kD, manufactured by Spectrum) and dialyzed overnight at 4°C in a 3L-volume beaker against PBS (a 10mM sodium phosphate buffer solution containing 0.15M NaCl, pH 7.3), while being stirred. As a result, white precipitates were observed in the dialysis bag. After dialysis, the sample was centrifuged under the conditions of 15000 rpm, 30 minutes, and 4°C to remove the precipitates (7), and filtered through a 0.22 µm filter membrane to remove the supernatant, resulting in a crude purified product (6).

As for the crude purified products of the three types of fusion peptides with the HIS tag, they were further applied to a Ni column (His GraviTrap, manufactured by GE Healthcare), captured, then eluted with 500 mM imidazole. The resulting eluate was dialyzed against PBS to produce a purified product.

### (Preparation Example 4) Preparation of Hybrid-E2s

The two types of polypeptides, trpE-his-G1E2s and trpE-his-G4E2s prepared in Preparation Example 3, produce a large quantity of precipitates during dialysis after solubilization in the aforementioned solubilization buffer. Therefore, by mixing each of them with trpE-G3E2s, which is less likely to produce precipitates, it was possible to purify them as heterodimers (Hybrid). For instance, trpE-G3E2s and trpE-his-G1E2s or trpE-his-G4E2s were solubilized from the ultrasonic disruption material (1) of Escherichia coli in the aforementioned solubilization buffer, mixed, and then dialyzed to purify them as heterodimers thereof (Hybrid-E2s).

### (4-1) Preparation of Hybrid-E2s (G3/G1-E2s)

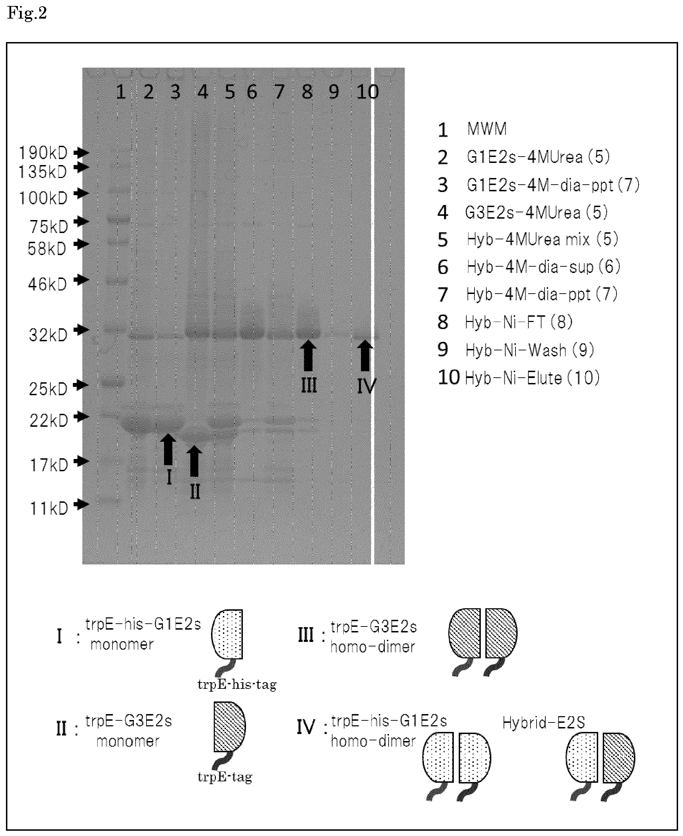

Firstly, the solubilized matter of trpE-his-G1E2s (G1E2s-4MUrea(5)) obtained in Preparation Example 3 and the solubilized matter of trpE-G3E2s (G3E2s-4MUrea(5)) were mixed in equal concentration (1:1) to obtain a mixture solution (Hyb-4MUrea mix(5)). Subsequently, the mixture solution was dialyzed overnight at 4°C in PBS, as in Preparation Example 3. After dialysis, the sample was centrifuged under the conditions of 15000 rpm, 30 minutes, and 4°C, the precipitate (Hyb-4M-dia-ppt(7)) was removed, and the supernatant (Hyb-4M-dia-sup(6)) was recovered. Next, the recovered supernatant was applied to a Ni column (His GraviTrap, manufactured by GE Healthcare). The pass-through fraction at this time (Hyb-Ni-FT(8)) was trpE-G3E2s without a HIS tag (Lane 8 (III) in Fig. 2 below). Subsequently, after washing with column washing buffer (PBS) (pass-through fraction: Hyb-Ni-Wash(9)), the eluate eluted with 500 mM imidazole was dialyzed against PBS to obtain the purified product (Hyb-Ni-Elute (10)).

Fig. 2 shows an electrophoretic photograph obtained by performing SDS-polyacrylamide gel electrophoresis on a molecular weight marker (MWM) and solutions or precipitates (5) to (10) obtained at each purification stage, where the precipitates were solubilized in 6M urea-containing PBS. Fig. 2 also shows the results of electrophoresis of the precipitate (G1E2s-4M-dia-ppt(7)) that occurred during the individual dialysis of trpE-his-G1E2s solubilized in the solubilization buffer (containing 4M urea) in Preparation Example 3. If trpE-his-G1E2s is present alone without forming a heterodimer, it is mostly removed as a precipitate through the aforementioned dialysis (Lane 3 (I) in Fig. 2). Therefore, the purified product obtained from the supernatant of the dialysis (Lane 10 (IV) in Fig. 2) contains a heterodimer of trpE-G3E2s and trpE-his-G1E2s (Hybrid-E2s (G3/G1-E2s)) and a dimer of trpE-his-G1E2s (homo-dimer). However, since trpE-his-G1E2s is mostly removed as a precipitate by the aforementioned dialysis, it can be said that the majority thereof is Hybrid-E2s. Note that it is considered that when trpE-his-G1E2s is mixed with trpE-G3E2s and dialyzed, trpE-G3E2s exerts a chaperone effect on trpE-his-G1E2s due to their contact and formation of the dimer.

### (4-2) Preparation of Hybrid-E2s (G3/G4-E2s)

Apart from using the solubilized matter of trpE-his-G4E2s (G4E2s-4MUrea(5)) prepared in Preparation Example 3 instead of the solubilized matter of trpE-his-G1E2s (G1E2s-4MUrea(5)), a purified product was obtained in the same manner as in Preparation Example 4-1.

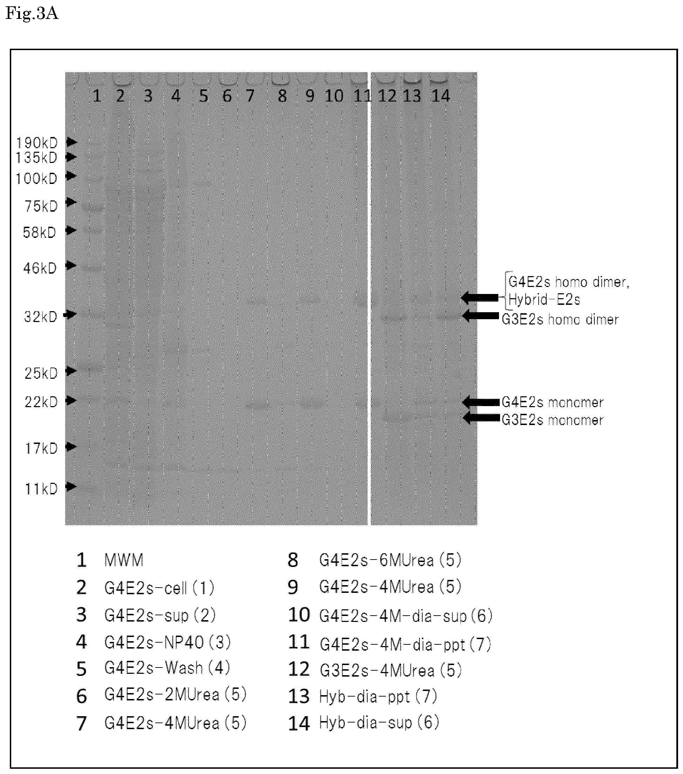

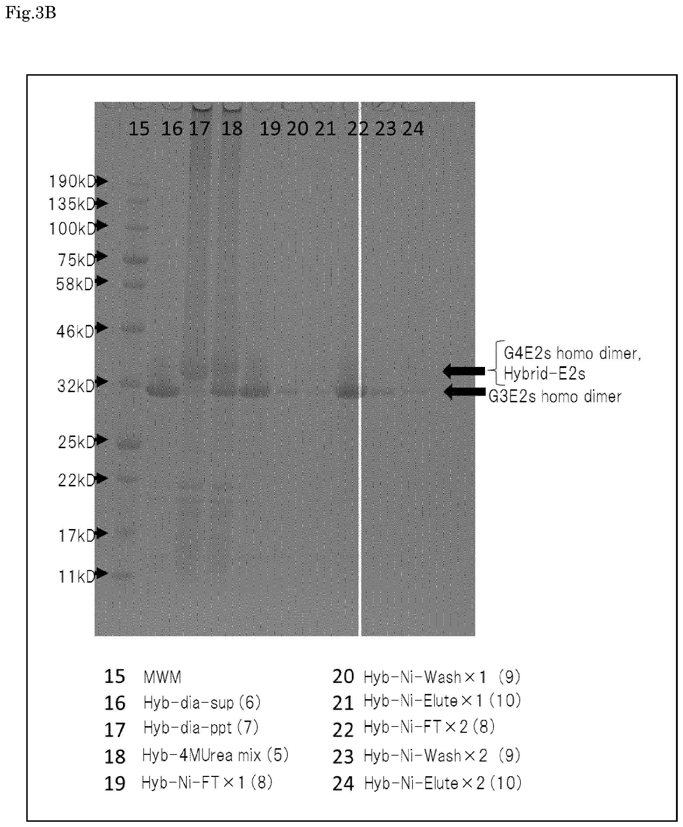

Figs. 3A and 3B show electrophoretic photographs obtained by performing SDS-polyacrylamide gel electrophoresis on a molecular weight marker (MWM) and solutions or precipitates obtained at each purification stage, where the precipitates were solubilized in 6M urea-containing PBS. Fig. 3A shows, from the left of the viewer, the molecular weight marker (MWM), ultrasonic disruption material of Escherichia coli of trpE-his-G4E2s (G4E2s-cell(1)) and the centrifuged supernatant thereof (G4E2s-sup(2)), the centrifuged supernatant after washing with a surfactant-containing washing liquid (G4E2s-NP40(3)) and the centrifuged supernatant after re-washing (G4E2s-Wash(4)), solubilized matter solubilized with a solubilization buffer (2M, 4M, 6M, or 4M urea, 25 mM Tris-HCl (pH 8.0), and 5 mM EDTA) (G4E2s-2M, 4M, 6M, 4MUrea(5)), a crude purified product after dialysis of the solubilized matter solubilized with a 4M urea-containing solubilization buffer (G4E2s-4M-dia-sup(6)) and the centrifugal precipitate thereof (G4E2s-4M-dia-ppt(7)), the solubilized matter of trpE-G3E2s solubilized with a 4M urea-containing solubilization buffer (G3E2s-4MUrea(5)), the centrifuged precipitate after dialysis of the mixture solution of the solubilized matter of trpE-his-G4E2s solubilized with a 4M urea-containing solubilization buffer and the solubilized matter of trpE-G3E2s solubilized with a 4M urea-containing solubilization buffer (Hyb-dia ppt(7)), and the crude purified product thereof (Hyb-dia sup(6)).

Fig. 3B shows, from the left of the viewer, the molecular weight marker (MWM), the aforementioned Hyb-dia-sup(6), the aforementioned Hyb-dia-ppt(7), a mixture solution of the solubilized matter of trpE-his-G4E2s solubilized with a 4M urea-containing solubilization buffer and the solubilized matter of trpE-G3E2s solubilized with a 4M urea-containing solubilization buffer (Hyb-4MUrea mix(5)), the Ni column pass-through fraction after dialysis of the above mixture solution (Hyb-Ni-FT(8)), the pass-through fraction during the washing of the Ni column (Hyb-Ni-Wash(9)), the purified product of the Ni column eluate (Hyb-Ni-Elute(10)), and those with double volumes of Lanes 19 to 21.

When trpE-his-G4E2s is dialyzed alone, most of it is recovered as a precipitate (lanes 10 and 11 in Fig. 3A). Therefore, as with the combination of trpE-G3E2s and trpE-his-G1E2s, the purified product contains the heterodimer of trpE-G3E2s and trpE-his-G4E2s (Hybrid-E2s (G3/G4-E2s)), and the dimer of trpE-his-G4E2s (homo-dimer), but it can be said that the majority thereof is Hybrid-E2s.

### (Preparation Example 5) Production of monoclonal antibodies recognizing G1E2s and G3E2s

### (5-1) Preparation of hybridoma

To prepare monoclonal antibodies that recognize trpE-his-G1E2s and trpE-his-G3E2s as antigen polypeptides, immunization was performed in mice as follows. Specifically, a mixed antigen of equal amounts of trpE-his-G1E2s and trpE-his-G3E2s, purified in Preparation Example 3, was administered intraperitoneally to BALB/c mice with 25 to 100 µg of TiterMax Gold (manufactured by Titer Max), an adjuvant. Two weeks later, additional immunization was performed similarly, and about two weeks later, a solution of the mixed antigen dissolved in PBS was administered into the tail vein as the final immunization.

Three days after the final immunization, the spleen was aseptically removed from the mice, and the spleen was broken down into individual cells using scissors and a metal mesh, then washed three times in RPMI-1640 medium. The mouse myeloma cell line SP2/0-Ag14 in the logarithmically growing period was washed three times in RPMI-1640 medium, and these cells were mixed with the aforementioned spleen cells at a cell ratio of 1:5. After centrifugation at 200 × g for 5 minutes, the supernatant was removed, and while gently mixing the precipitated cell clumps, 1 mL of RPMI-1640 medium containing 50% polyethylene glycol (PEG) 4000 (manufactured by Merck) was slowly added, and then 10 mL of RPMI-1640 medium was added to fuse the cells.

The obtained fusion cells were centrifuged at 200 × g for 5 minutes to remove PEG, then suspended in RPMI-1640 medium containing 10% fetal bovine serum and hypoxanthine-aminopterin-thymidine (HAT), and seeded onto a 96-well cell culture plate. After approximately 10 days of cultivation, only the hybridomas were allowed to proliferate, and then clones producing monoclonal antibodies specifically reacting to the above mixed antigens were searched by the ELISA method, yielding hybridomas reacting to the mixed antigens. The hybridomas were cloned into single clones by the limiting dilution method, establishing the desired hybridomas of six strains (A505, A519, A906, A907, A924, and A926).

Each established hybridoma strain was cultured for 2 to 3 weeks at 37°C in a 5% carbon dioxide environment using serum-free medium (Hybridoma-SFM, manufactured by GIBCO). The culture fluid after culture was subjected to affinity chromatography using a Protein A Sepharose column, and the monoclonal antibodies (A505, A519, A924, and A926) produced in the culture fluid were purified.

Subsequently, the ELISA reactivity was evaluated when trpE-his-G1E2s (G1E2s antigen) and trpE-his-G3E2s (G3E2s antigen) were each used as an antigen polypeptide. First, each antigen polypeptide purified in Preparation Example 3 was diluted in PBS (10 mM sodium phosphate buffer containing 0.15M NaCl (pH7.3)) so that the final concentration was 0.5 µg/mL, and 100 µL per well was dispensed into a 96-well microplate (manufactured by Nunc). Next, it was allowed to stand overnight at 4°C, each well was washed twice using 0.35 mL of PBS. Then, 0.35 mL of blocking solution (PBS containing 0.5% casein-Na) was added to each well, and it was further allowed to stand at room temperature for 3 hours. The blocking solution was removed, and then the microplate was vacuum dried to prepare the ELISA plate, and stored at 4°C until use.

Next, to each well of the aforementioned ELISA plate, 100 µL of antibody reaction solution containing 0.2 µg/mL of each monoclonal antibody obtained above in Reaction Solution Buffer 1 (100mM sodium phosphate buffer containing 0.2M NaCl, 1% BSA, 0.07% casein-Na (pH 7.3)) was added, and after keeping it at room temperature for 60 minutes, it was washed five times with PBS-T (PBS containing 0.05% Tween 20). Then, to each well, labeled body solution containing POD (peroxidase)-labelled anti-mouse IgG antibodies (manufactured by Jackson ImmunoResearch) in the aforementioned Reaction Solution Buffer 1 was added and kept at room temperature for 60 minutes, and then washed five times with PBS-T. Then, to each well, 100 µL of mixed solution of ELISA POD Substrate TMB Kit (manufactured by Nacalai Tesque) was added and reacted at room temperature in the dark for 30 minutes. After the reaction, a 2N sulfuric acid solution was added to each well by 100 µL, and the absorbance at a wavelength of 630 nm (OD630) was used as a control to measure the absorbance at a wavelength of 450 nm (OD450), and the ELISA reactivity was evaluated.

Table 2 below shows the results of ELISA reactivity when G1E2s antigen and G3E2s antigen were each used as an antigen polypeptide. In Table 2, "-" indicates no reactivity (OD450: less than 0.500), "+" indicates sufficient reactivity (OD450: 1.000 or more and less than 3.000), and "++" indicates strong reactivity (OD450: 3.000 or more). As shown in Table 2, reactivity was recognized for all except A505 when G1E2s was used as an antigen polypeptide. Meanwhile, when G3E2s was used as an antigen polypeptide, reactivity was recognized for A505, A519, A924, and A926.

### (5-2) Isotypes of the acquired monoclonal antibodies

Each hybridoma strain established in (5-1) was cultured for 2 to 3 weeks at 37°C in a 5% carbon dioxide environment using serum-free medium (Hybridoma-SFM, manufactured by GIBCO). The culture fluid after culture was subjected to affinity chromatography using a Protein A Sepharose column, and the monoclonal antibodies (A505, A519, A924, and A926) produced in the culture fluid were purified. Then, the subclasses of the purified monoclonal antibodies were identified using an isotype typing kit (manufactured by Zymed) that uses anti-mouse Ig isotype antibodies. Table 2 below shows the results. As shown in Table 2, all isotypes were IgG, and except for A505, which is subclass IgG2b, it was confirmed that all were subclass IgGl.

**[Table 2]**

| Hybridoma Strain Monoclonal Antibody | Antigen | | | | Isotype (Subclass) |
|---|---|---|---|---|---|
| | G1E2s | (Reactivity) | G3E2s | (Reactivity) | |
| A505 | 0.426 | - | 3.290 | ++ | IgG2b |
| A519 | 2.475 | + | 3.268 | ++ | IgGl |
| A906 | 3.548 | ++ | 0.013 | - | IgGl |
| A907 | 3.340 | ++ | 0.334 | - | IgGl |
| A924 | 2.398 | + | 2.360 | + | IgG1 |
| A926 | 3.083 | ++ | 2.795 | + | IgGl |

### (Preparation Example 6) Preparation of biotinylated E2s antigens and biotinylated monoclonal antibodies

To biotinylate G1E2s antigen, G3E2s antigen, and Hybrid-E2s antigen, along with each monoclonal antibody, biotin reagent (Sulfo-NHS-LC-Biotin, manufactured by Thermo Fisher Scientific) was used to modify each of the antigens and antibodies with biotin. Specifically, first, immediately before use, 1 mg of biotin reagent was dissolved in 180 µL of ultrapure water to prepare a 10mM biotin solution. Subsequently, the trpE-his-G1E2s (G1E2s antigen) and trpE-his-G3E2s (G3E2s antigen) prepared in Preparation Example 3, and the Hybrid-E2s (G3/G1-E2s) (Hybrid-E2s antigen, Hyb-Ag) prepared in Preparation Example 4, as well as each monoclonal antibody prepared in Preparation Example 5, were dissolved in 1 mL of PBS, and 1 mg of each was mixed with 66.7 µL of the above 10mM biotin solution, and kept at room temperature for 30 minutes. Subsequently, PD-10 was used to desalt the unreacted biotin reagent with PBS, and the biotin-modified E2s antigens (biotinylated E2s antigens) and each monoclonal antibody (biotinylated antibody) were purified.

### (Test Example 1) Evaluation of Hybrid-E2s (G3/G1-E2s)

In Table 2, evaluations were conducted on the Hybrid-E2s (G3/G1-E2s) (Hybrid-E2s antigen) prepared in Preparation Example 4, using sandwich ELISA with monoclonal antibody A505, which shows no reactivity to the G1E2s antigen, and monoclonal antibody A907, which shows no reactivity to the G3E2s antigen; sandwich ELISA with monoclonal antibody A906, which shows no reactivity to the G3E2s antigen, and monoclonal antibody A924, which has reactivity to both the G1E2s antigen and the G3E2s antigen; sandwich ELISA with monoclonal antibody A926, which has reactivity to both the G1E2s antigen and the G3E2s antigen, and monoclonal antibody A907, which shows no reactivity to the G3E2s antigen; and sandwich ELISA with monoclonal antibodies A924 and A926, which have reactivity to both the G1E2s antigen and the G3E2s antigen.

First, as with Example 1 below, monoclonal antibodies A907 and A924 were each immobilized on a 96-well microplate (0.5 µg per well). Subsequently, to each well, 90 µL of the aforementioned Reaction Solution Buffer 1 was added, along with 10 µL of trpE-his-G1E2s (G1E2s antigen) or trpE-his-G3E2s (G3E2s antigen) prepared in Preparation Example 3, or Hybrid-E2s antigen prepared in Preparation Example 4, each at 1 µg/mL (final antigen concentration: 0.1 µg/mL each), and reacted at room temperature for 1 hour. After the reaction, each well was washed five times with 0.35 mL of washing liquid (PBS containing 0.05% Tween 20). Then, 100 µL of the labeled body solution containing each biotinylated monoclonal antibody (0.2 µg/mL) prepared in Preparation Example 6 in the aforementioned Reaction Solution Buffer 1 was added to each well of the plate in the combination shown in Table 3 below, and reacted at room temperature for 1 hour. Subsequently, after washing five times with the washing liquid, 100 µL of labeled body solution, prepared by diluting streptavidin labeled with POD (500 units/mL) (SA-POD, manufactured by Roche) 5000 times, was added to each well, and reacted at room temperature for 30 minutes. After washing five times with the washing liquid, 100 µl of mixed solution of ELISA POD Substrate TMB Kit (manufactured by Nacalai Tesque) was added and reacted in the dark at room temperature for 30 minutes. After the reaction, 100 µL of 2N sulfuric acid solution was added to each well, and the absorbance at a wavelength of 630 nm (OD630) was used as a control to measure the absorbance at a wavelength of 450 nm (OD450). The value calculated by the formula: OD450 - OD630 was taken as the measured value and is shown in Table 3 below. Note that Table 3 also shows the S/N value of each measured value (the value obtained by dividing each measured value by the value OD450 - OD630 without added antigen (blank)). In the "(Reactivity)" columns in Table 3, "-" indicates no reactivity (measured value: less than 0.100), "+" indicates sufficient reactivity (measured value: 0.100 or more and less than 1.000), and "++" indicates strong reactivity (measured value: 1.000 or more).

**[Table 3]**

| Antigen | Label Antibody | Immobilized Antibody | | | | S/N | |
|---|---|---|---|---|---|---|---|
| | | A907 | (Reactivity) | A924 | (Reactivity) | A907 | A924 |
| G3E2s | A505 | 0.653 | + | - | | 54.4 | - |
| | A906 | - | | 0.014 | - | - | 1.2 |
| | A926 | 0.510 | + | 1.179 | ++ | 42.5 | 98.3 |
| G1E2s | A505 | 0.016 | - | - | | 1.3 | - |
| | A906 | - | | 0.314 | + | - | 26.2 |
| | A926 | 0.936 | + | 1.071 | ++ | 78.0 | 89.3 |
| Hyb-Ag | A505 | 0.791 | + | - | | 65.9 | - |
| | A906 | - | | 0.287 | + | - | 23.9 |
| | A926 | 1.236 | ++ | 1.519 | ++ | 103.0 | 126.6 |
| blank | - | 0.012 | | 0.012 | | 1.0 | 1.0 |

As shown in Table 3, both the sandwich ELISA with monoclonal antibodies A505 and A907, which is a combination capable of specifically reacting only with the G3E2s antigen, and the sandwich ELISA with monoclonal antibodies A906 and A924, which is a combination capable of specifically reacting with the G1E2s antigen, reacted with the Hybrid-E2s antigen. This confirmed that the Hybrid-E2s antigen was composed of the G3E2s antigen and the G1E2s antigen.

### (Test Example 2) Confirmation of reactivity by each measurement system for HEV positive and negative samples

### (Example 1) IgM measurement system

Anti-human IgM antibodies commercially available or produced by a conventionally known method were diluted in PBS (10 mM sodium phosphate buffer containing 0.15M NaCl (pH7.3)) so that the final concentration was 5 µg/mL, and 100 µL per well was dispensed into a 96-well microplate (manufactured by Nunc). Next, it was allowed to stand overnight at 4°C, each well was washed twice using 0.35 mL of PBS. Then, 0.35 mL of blocking solution (PBS containing 0.5% casein-Na) was added to each well, and it was further allowed to stand at room temperature for 3 hours. The blocking solution was removed, and then the microplate was vacuum dried to prepare an anti-human IgM antibody-immobilized plate, and stored at 4°C until use.

This was used to measure the HEV IgM antibody titers in a group of samples (16 samples) composed of human plasma containing hepatitis E patient plasma purchased from TRINA. Specifically, to the anti-human IgM antibody-immobilized plate, 90 µL of Reaction Solution Buffer 2, containing 50 mM Tris-HCl (pH 7.2), 150 mM NaCl, 1 mM EDTA, 2% BSA, 20 ppm KLG, and 2% gelatin, and 10 µL of each sample, diluted 10-fold with Reaction Solution Buffer 2, were added, followed by reaction at room temperature for 1 hour. After the reaction, each well was washed five times with 0.35 mL of washing liquid (PBS containing 0.05% Tween 20). Thereafter, to each well of the anti-human IgM antibody-immobilized plate, 100 µL each of three types of biotinylated E2s antigens (1 mg/mL) prepared in Preparation Example 6, each diluted 5000 times (diluent: Reaction Solution Buffer 2), was added, and reacted at room temperature for 1 hour. Subsequently, after washing with the washing liquid, 100 µL of labeled body solution, prepared by diluting alkaline phosphatase (ALP) -labeled streptavidin (0.2 mg/mL) (SA-ALP, manufactured by Thermo Fisher Scientific) 5000 times, was added to each well, and reacted at room temperature for 30 minutes. Subsequently, after washing with the washing liquid, 100 µL of Lumipulse (registered trademark) substrate solution (manufactured by Fujirebio), containing AMPPD (3-(2'-spiroadamantane)-4-methoxy-4-(3'-phosphoryloxy)phenyl-1,2-dioxetane disodium salt) was added, and reacted at room temperature in the dark for 20 minutes. After the reaction, Lumipulse (registered trademark) L-2400 (manufactured by Fujirebio) was used to measure the luminescence intensity (counts) of light with a maximum absorption at a wavelength of 463 nm, which is emitted by the decomposition of AMPPD due to the catalytic action of ALP, thus obtaining the measurement results (HEV IgM antibody titers in the samples). Table 4 below shows the measurement results for each sample. Note that the results shown in Table 4 are the average values of duplicate measurements.

### (Comparative Example 1) IgM measurement system

A commercially available anti-HEV-IgM ELISA kit that uses amino acids 394 to 606 encoded by the ORF2 of the HEV genotype 1 gene (DDBJ Accession No.: D11092) as a recombinant HEV antigen polypeptide was employed to measure the same group of samples as in Example 1 by the ELISA method using an anti-IgM antibody-immobilized plate as a capture body and the recombinant HEV antigen polypeptide labeled with POD as the labeled body. As to the coloration caused by the TMBZ oxidant liberated in the reaction of POD with hydrogen peroxide and tetramethylbenzidine (TMBZ) substrate, the absorbance at a wavelength of 450 nm (OD450) was measured with the absorbance at a wavelength of 630 nm (OD630) as a control. The value calculated by the formula: OD450 - OD630 was taken as the HEV IgM antibody titer in the sample.

### (Comparative Example 2) IgA measurement system

A commercially available anti-HEV-IgA ELISA kit that uses amino acids 111 to 660 encoded by the ORF2 of the HEV genotype 4 gene (DDBJ/GenBank/EMBL Accession No.: AB082545) as a recombinant HEV antigen polypeptide was employed to measure the same group of samples as in Example 1 by the ELISA method using a plate immobilized with the recombinant HEV antigen polypeptide as a capture body and an anti-human IgA antibody labeled with POD as the labeled body. As to the coloration caused by the TMBZ oxidant liberated in the reaction of POD with hydrogen peroxide and tetramethylbenzidine (TMBZ) substrate, the absorbance at a wavelength of 450 nm (OD450) was measured with the absorbance at a wavelength of 630 nm (OD630) as a control. The value calculated by the formula: OD450 - OD630 was taken as the HEV IgA antibody titer in the sample.

The measurement results (three types) measured in Example 1 and the measurement results measured in Comparative Examples 1 and 2 are summarized in Table 4 below. The cutoff value of Comparative Example 1 was calculated by the formula: (average value of the negative control (sample without IgM class anti-HEV antibody)) + 0.26, and the cutoff value of Comparative Example 2 was calculated by the formula: (average value of positive control (sample with IgA class anti-HEV antibody) - average value of negative control (sample without IgA class anti-HEV antibody)) × 0.5. As to the cutoff value of Example 1, the average value + 8SD of a group of samples (negative samples: n = 40) composed of human plasma free of hepatitis E patient plasma purchased from TRINA was separately applied. In Example 1 of Table 4, "-" indicates negative (less than the cutoff value), "+" indicates positive (equal to or greater than the cutoff value and less than the cutoff value × 3), "++" indicates strong positive (equal to or greater than the cutoff value × 3 and less than the cutoff value × 6), "+++" indicates particularly strong positive (equal to or greater than the cutoff value × 6 and less than the cutoff value × 30), and "++++" indicates extremely strong positive (equal to or greater than the cutoff value × 30) (the same applies in Example 2 in the following Table 5). Also, in Comparative Examples 1 and 2 of Table 4, "-" indicates negative (less than the cutoff value), "+" indicates positive (equal to or greater than the cutoff value and less than the cutoff value × 3), "++" indicates strong positive (equal to or greater than the cutoff value × 3) (the same applies in Comparative Examples 1 and 2 in the following Table 5).

**[Table 4]**

| Sampe No. | IgM (Example 1) | | | | | | IgM (Comparative Example 1) | (Reactivity) | IgA (Comparative Example 2) | (Reactivity) |
|---|---|---|---|---|---|---|---|---|---|---|
| | G1E2s | (Reactivity) | G3E2s | (Reactivity) | Hyb-Ag | (Reactivity) | | | | |
| 1 | 104209 | +++ | 14335 | + | 174075 | ++ | 0.494 | + | 1.162 | + |
| 2 | 137330 | +++ | 149402 | +++ | 500948 | +++ | 0.885 | ++ | 1.277 | + |
| 3 | 3294 | - | 2043 | - | 3648 | - | 0.005 | - | 0.032 | - |
| 4 | 23396 | + | 18870 | + | 98579 | + | 0.079 | - | 0.321 | - |
| 5 | 9904 | - | 2830 | - | 21129 | - | 0.026 | - | 0.049 | - |
| 6 | 57374 | ++ | 20765 | + | 134042 | ++ | 0.321 | + | 1.055 | + |
| 7 | 128424 | +++ | 127185 | +++ | 564421 | +++ | 0.376 | + | 0.910 | - |
| 8 | 18727 | + | 12651 | + | 57582 | + | 0.160 | - | 0.139 | - |
| 9 | 109687 | +++ | 508577 | ++++ | 1220480 | ++++ | 0.784 | + | 1.038 | + |
| 10 | 57589 | ++ | 7228 | - | 143026 | ++ | 0.171 | - | 0.400 | - |
| 11 | 100010 | +++ | 56939 | ++ | 332903 | +++ | 0.853 | ++ | 0.625 | - |
| 12 | 45284 | + | 19516 | + | 110548 | ++ | 0.236 | - | 0.346 | - |
| 13 | 47452 | + | 14399 | + | 103624 | ++ | 0.110 | - | 1.528 | + |
| 14 | 15792 | - | 5782 | - | 47688 | + | 0.040 | - | 0.276 | - |
| 15 | 293161 | +++ | 267863 | +++ | 837414 | +++ | 1.009 | ++ | 0.752 | - |
| 16 | 6001 | - | 3541 | - | 25626 | - | 0.013 | - | 0.200 | - |
| Cutoff Value | 16621 | | 11344 | | 32865 | | 0.262 | | 0.976 | |

In Table 4, three samples, No. 3, No. 5, and No. 16, were HEV infection-negative samples. All other samples reacted in some measurement system. First, compared with the IgM measurement system, the reactivity was higher in any of the samples when using the G1E2s antigen, G3E2s antigen, or Hybrid-E2s antigen in the measurement system of Example 1, compared to the measurement system of Comparative Example 1. In particular, 6 samples, No. 4, No. 8, No. 10, No. 12, No. 13, and No. 14, were positive in any of Example 1, whereas in Comparative Example 1, they turned out to be negative (false negatives).

Furthermore, even within Example 1, compared to when the G1E2s antigen or G3E2s antigen was used alone, the use of the Hybrid-E2s antigen, which is a heterodimer thereof, showed a higher reactivity to samples that were negative with at least one of the antigens alone (No. 10), and further, it also showed reactivity to the test that was negative with either antigen alone (No. 14). This confirmed a synergistic effect of detecting positive samples incapable of detection with the individual antigen polypeptide.

Next, compared with the IgA measurement system, the reactivity was higher in any of the samples when using the G1E2s antigen, G3E2s antigen, or Hybrid-E2s antigen in the measurement system of Example 1, compared to the measurement system of Comparative Example 2. In particular, 8 samples, No. 4, No. 7, No. 8, and No. 10 to 12, No. 14, and No. 15, were positive in any of Example 1, whereas in Comparative Example 2, they turned out to be negative (false negatives). Note that since IgA arises from a different immune mechanism from IgM, direct comparison is difficult, and it is considered to show a reactivity that is intermediate between IgM and IgG.

### (Example 2) IgG measurement system

The trpE-his-G3E2s (G3E2s antigen) prepared in Preparation Example 3 was diluted in PBS so that the final concentration was 0.5 µg/mL, and 100 µL per well was dispensed into a 96-well microplate. Next, it was allowed to stand overnight at 4°C, each well was washed twice using 0.35 mL of PBS. Then, 0.35 mL of the same blocking solution as in Example 1 was added to each well, and it was further allowed to stand at room temperature for 3 hours. The blocking solution was removed, and then the microplate was vacuum dried to prepare a G3E2s antigen-immobilized plate, and stored at 4°C until use.

This was used to measure the HEV IgG antibody titer in the same sample group as in Example 1. Specifically, to the G3E2s antigen-immobilized plate, 90 µL of the aforementioned Reaction Solution Buffer 2 and 10 µL of each sample, diluted 10-fold with Reaction Solution Buffer 2, were added, followed by reaction at room temperature for 1 hour. After the reaction, 0.35 mL of the same washing liquid as in Example 1 was used to wash each well five times. Thereafter, to each well, 100 µL of POD-labeled anti-human IgG antibody (1 mg/mL), diluted 5000 times (diluent: Reaction Solution Buffer 2), was added, and reacted at room temperature for 1 hour. Subsequently, after washing five times with the washing liquid, to each well, 100 µL of mixed solution of ELISA POD Substrate TMB Kit (manufactured by Nacalai Tesque) was added and reacted at room temperature in the dark for 30 minutes. After the reaction, 100 µL of 2N sulfuric acid solution was added to each well, and the absorbance at a wavelength of 630 nm (OD630) was used as a control to measure the absorbance at a wavelength of 450 nm (OD450), and the value calculated by the formula: OD450 - OD630 was taken as the HEV IgG antibody titer in the sample.

Table 5 below shows the results measured in Example 2. The cutoff value of Example 2 was set at approximately 10 times (0.400) in S/N ratio, using as a control the average value of a group of samples (negative samples: n = 40) composed of human plasma free of hepatitis E patient plasma purchased separately from TRINA. Table 5 also shows the results of Comparative Examples 1 and 2 for each sample.

**[Table 5]**

| Sample No. | IgG (Example 2) | | IgM (Comparative Example 1) | (Reactivity) | IgA (Comparative Example 2) | (Reactivity) |
|---|---|---|---|---|---|---|
| | G3E2s | (Reactivity) | | | | |
| 1 | 0.453 | + | 0.494 | + | 1.162 | + |
| 2 | 1.373 | ++ | 0.885 | ++ | 1.277 | + |
| 3 | 0.200 | - | 0.005 | - | 0.032 | - |
| 4 | 0.418 | + | 0.079 | - | 0.321 | - |
| 5 | 0.315 | - | 0.026 | - | 0.049 | - |
| 6 | 0.699 | + | 0.321 | + | 1.055 | + |
| 10 | 0.104 | - | 0.171 | - | 0.400 | - |
| 13 | 0.547 | + | 0.110 | - | 1.528 | + |
| 14 | 0.103 | - | 0.040 | - | 0.276 | - |
| 15 | 2.801 | +++ | 1.009 | ++ | 0.752 | - |
| 16 | 0.238 | - | 0.013 | - | 0.200 | - |
| Cutoff Value | 0.400 | | 0.262 | | 0.976 | |

As with the results shown in Table 4, three samples, No. 3, No. 5, and No. 16, were HEV infection-negative samples, and furthermore, HEV-IgG negative samples were confirmed in 2 samples, No. 10 and No. 14. All other samples reacted in some measurement system. As shown in Table 5, even in the IgG measurement system, the measurement system of Example 2 using the G3E2s antigen showed higher reactivity than the measurement system of Comparative Example 1, and also had higher reactivity compared to Comparative Example 2 using the antigen as a capture body in a similar manner to Example 2.

### [Industrial Applicability]

The present invention makes it possible to provide a method for detecting hepatitis E virus infections with higher sensitivity than conventional methods, allowing for detection of anti-hepatitis E virus antibodies, and an antigen polypeptide and kit for detecting hepatitis E virus infections used therefor.

### [Sequence Listing Free Text]

SEQ ID NO:5
   <223> reverse primer G3AS1
SEQ ID NO:6
   <223> primer G3S1
SEQ ID NO:7
   <223> primer G3S2
SEQ ID NO:8
   <223> primer G3AS2
SEQ ID NO:9
   <223> primer G3E2s-his452-S
SEQ ID NO:10
   <223> primer G3E2s-617-AS
SEQ ID NO:11
   <223> primer G3E2s-452-S
SEQ ID NO:12
   <223> G1E2s 394-617
SEQ ID NO:13
   <223> primer G1E2s-his452-S
SEQ ID NO:14
   <223> primer G1E2s-617-AS
SEQ ID NO:15
   <223> reverse primer G4AS1
SEQ ID NO:16
   <223> primer G4S1
SEQ ID NO:17
   <223> primer G4S2
SEQ ID NO:18
   <223> primer G4AS2
SEQ ID NO:19
   <223> primer G4E2s-his445-S
SEQ ID NO:20
   <223> primer G4E2s-606-AS

## Claims

1. A method for detecting hepatitis E virus infection, comprising a detection step of detecting an anti-hepatitis E virus antibody in a sample by using, as an antigen polypeptide, a dimer consisting of any one polypeptide selected from the group consisting of the following (3a) to (3c), and any one polypeptide selected from the group consisting of the following (1a) to (1c), (2a) to (2c), and (4a) to (4c):
(1a) a polypeptide consisting of an amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(1b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(1c) a polypeptide consisting of an amino acid sequence having 80% or more homology with the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(2a) a polypeptide consisting of an amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(2b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(2c) a polypeptide consisting of an amino acid sequence having 80% or more homology with the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(3a) a polypeptide consisting of an amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(3b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(3c) a polypeptide consisting of an amino acid sequence having 80% or more homology with the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(4a) a polypeptide consisting of an amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4;
(4b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4; and
(4c) a polypeptide consisting of an amino acid sequence having 80% or more homology with the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4.

2. The method for detecting hepatitis E virus infection according to claim 1, wherein an isotype of the anti-hepatitis E virus antibody is IgM.

3. The method for detecting hepatitis E virus infection according to claim 1, wherein
the detection step comprises a step of bringing the sample into contact with a capture body and a labeled body, and
the capture body comprises a water insoluble carrier and a probe molecule capable of binding to the anti-hepatitis E virus antibody, and
the labeled body comprises a labeling substance and the antigen polypeptide.

4. An antigen polypeptide for detecting hepatitis E virus infection according to the method for detecting hepatitis E virus infection according to claim 1, which is a dimer consisting of any one polypeptide selected from the group consisting of the following (3a) to (3c), and any one polypeptide selected from the group consisting of the following (1a) to (1c), (2a) to (2c), and (4a) to (4c):
(1a) a polypeptide consisting of an amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(1b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(1c) a polypeptide consisting of an amino acid sequence having 80% or more homology with the amino acid sequence which contains amino acids located at position 28 to 175 in the amino acid sequence set forth in SEQ ID NO: 1 and which has a full length of 200 residues or less that are contained in the amino acid sequence set forth in SEQ ID NO: 1;
(2a) a polypeptide consisting of an amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(2b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(2c) a polypeptide consisting of an amino acid sequence having 80% or more homology with the amino acid sequence which contains amino acids located at position 28 to 175 in the amino acid sequence set forth in SEQ ID NO: 2 and which has a full length of 200 residues or less that are contained in the amino acid sequence set forth in SEQ ID NO: 2;
(3a) a polypeptide consisting of an amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(3b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(3c) a polypeptide consisting of an amino acid sequence having 80% or more homology with the amino acid sequence which contains amino acids located at position 28 to 175 in the amino acid sequence set forth in SEQ ID NO: 3 and which has a full length of 200 residues or less that are contained in the amino acid sequence set forth in SEQ ID NO: 3;
(4a) a polypeptide consisting of an amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4;
(4b) a polypeptide consisting of an amino acid sequence in which one or more amino acids are substituted, deleted, inserted, and/or added in the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4; and
(4c) a polypeptide consisting of an amino acid sequence having 80% or more homology with the amino acid sequence which contains amino acids located at position 28 to 175 in the amino acid sequence set forth in SEQ ID NO: 4 and which has a full length of 200 residues or less that are contained in the amino acid sequence set forth in SEQ ID NO: 4.

5. The antigen polypeptide according to claim 4, which is a labeled body labeled with a labeling substance.

6. A kit for detecting hepatitis E virus infection according to the method for detecting hepatitis E virus infection according to claim 1, comprising the antigen polypeptide according to claim 4 or 5.

7. The kit for detecting hepatitis E virus infection according to claim 6, further comprising a capture body comprising a water insoluble carrier and a probe molecule capable of binding to an anti-hepatitis E virus antibody.

## Patentansprüche

1. Verfahren zum Nachweisen einer Hepatitis-E-Virus-Infektion, umfassend einen Nachweisschritt des Nachweisens eines Anti-Hepatitis-E-Virus-Antikörpers in einer Probe unter Verwendung, als ein Antigen-Polypeptid, eines Dimers bestehend aus einem beliebigen Polypeptid ausgewählt aus der Gruppe bestehend aus den folgenden (3a) bis (3c) und einem beliebigen Polypeptid ausgewählt aus der Gruppe bestehend aus den folgenden (1a) bis (1c), (2a) bis (2c) und (4a) bis (4c):
(1a) ein Polypeptid bestehend aus einer Aminosäuresequenz, die Aminosäuren an Positionen 28 bis 175 der in SEQ ID NO: 1 angegebenen Aminosäuresequenz enthält und die eine Gesamtlänge von 200 oder weniger Resten aufweist, die in der in SEQ ID NO: 1 angegebenen Aminosäuresequenz enthalten sind;
(1b) ein Polypeptid bestehend aus einer Aminosäuresequenz, bei der eine oder mehrere Aminosäuren in der Aminosäuresequenz substituiert, deletiert, eingefügt und/oder hinzugefügt sind, die Aminosäuren an Positionen 28 bis 175 der in SEQ ID NO: 1 angegebenen Aminosäuresequenz enthält und die eine Gesamtlänge von 200 oder weniger Resten aufweist, die in der in SEQ ID NO: 1 angegebenen Aminosäuresequenz enthalten sind;
(1c) ein Polypeptid bestehend aus einer Aminosäuresequenz, die eine Homologie von 80 % oder mehr mit bzw. zu der Aminosäuresequenz aufweist, die Aminosäuren an Positionen 28 bis 175 der in SEQ ID NO: 1 angegebenen Aminosäuresequenz enthält und die eine Gesamtlänge von 200 oder weniger Resten aufweist, die in der in SEQ ID NO: 1 angegebenen Aminosäuresequenz enthalten sind;
(2a) ein Polypeptid bestehend aus einer Aminosäuresequenz, die Aminosäuren an Positionen 28 bis 175 der in SEQ ID NO: 2 angegebenen Aminosäuresequenz enthält und die eine Gesamtlänge von 200 oder weniger Resten aufweist, die in der in SEQ ID NO: 2 angegebenen Aminosäuresequenz enthalten sind;
(2b) ein Polypeptid bestehend aus einer Aminosäuresequenz, bei der eine oder mehrere Aminosäuren in der Aminosäuresequenz substituiert, deletiert, eingefügt und/oder hinzugefügt sind, die Aminosäuren an Positionen 28 bis 175 der in SEQ ID NO: 2 angegebenen Aminosäuresequenz enthält und die eine Gesamtlänge von 200 oder weniger Resten aufweist, die in der in SEQ ID NO: 2 angegebenen Aminosäuresequenz enthalten sind;
(2c) ein Polypeptid bestehend aus einer Aminosäuresequenz, die eine Homologie von 80 % oder mehr mit bzw. zu der Aminosäuresequenz aufweist, die Aminosäuren an Positionen 28 bis 175 der in SEQ ID NO: 2 angegebenen Aminosäuresequenz enthält und die eine Gesamtlänge von 200 oder weniger Resten aufweist, die in der in SEQ ID NO: 2 angegebenen Aminosäuresequenz enthalten sind;
(3a) ein Polypeptid bestehend aus einer Aminosäuresequenz, die Aminosäuren an Positionen 28 bis 175 der in SEQ ID NO: 3 angegebenen Aminosäuresequenz enthält und die eine Gesamtlänge von 200 oder weniger Resten aufweist, die in der in SEQ ID NO: 3 angegebenen Aminosäuresequenz enthalten sind;
(3b) ein Polypeptid bestehend aus einer Aminosäuresequenz, bei der eine oder mehrere Aminosäuren in der Aminosäuresequenz substituiert, deletiert, eingefügt und/oder hinzugefügt sind, die Aminosäuren an Positionen 28 bis 175 der in SEQ ID NO: 3 angegebenen Aminosäuresequenz enthält und die eine Gesamtlänge von 200 oder weniger Resten aufweist, die in der in SEQ ID NO: 3 angegebenen Aminosäuresequenz enthalten sind;
(3c) ein Polypeptid bestehend aus einer Aminosäuresequenz, die eine Homologie von 80 % oder mehr mit bzw. zu der Aminosäuresequenz aufweist, die Aminosäuren an Positionen 28 bis 175 der in SEQ ID NO: 3 angegebenen Aminosäuresequenz enthält und die eine Gesamtlänge von 200 oder weniger Resten aufweist, die in der in SEQ ID NO: 3 angegebenen Aminosäuresequenz enthalten sind;
(4a) ein Polypeptid bestehend aus einer Aminosäuresequenz, die Aminosäuren an Positionen 28 bis 175 der in SEQ ID NO: 4 angegebenen Aminosäuresequenz enthält und die eine Gesamtlänge von 200 oder weniger Resten aufweist, die in der in SEQ ID NO: 4 angegebenen Aminosäuresequenz enthalten sind;
(4b) ein Polypeptid bestehend aus einer Aminosäuresequenz, bei der eine oder mehrere Aminosäuren in der Aminosäuresequenz substituiert, deletiert, eingefügt und/oder hinzugefügt sind, die Aminosäuren an Positionen 28 bis 175 der in SEQ ID NO: 4 angegebenen Aminosäuresequenz enthält und die eine Gesamtlänge von 200 oder weniger Resten aufweist, die in der in SEQ ID NO: 4 angegebenen Aminosäuresequenz enthalten sind; und
(4c) ein Polypeptid bestehend aus einer Aminosäuresequenz, die eine Homologie von 80 % oder mehr mit bzw. zu der Aminosäuresequenz aufweist, die Aminosäuren an Positionen 28 bis 175 der in SEQ ID NO: 4 angegebenen Aminosäuresequenz enthält und die eine Gesamtlänge von 200 oder weniger Resten aufweist, die in der in SEQ ID NO: 4 angegebenen Aminosäuresequenz enthalten sind.

2. Verfahren zum Nachweisen einer Hepatitis-E-Virus-Infektion nach Anspruch 1, wobei ein Isotyp des Anti-Hepatitis-E-Virus-Antikörpers IgM ist.

3. Verfahren zum Nachweisen einer Hepatitis-E-Virus-Infektion nach Anspruch 1, wobei
der Nachweisschritt einen Schritt des Inkontaktbringens der Probe mit einem Fangkörper und einem markierten Körper umfasst, und
der Fangkörper einen wasserunlöslichen Träger und ein Probenmolekül umfasst, das in der Lage ist, an den Anti-Hepatitis-E-Virus-Antikörper zu binden, und
der markierte Körper eine Markierungssubstanz und das Antigen-Polypeptid umfasst.

4. Antigen-Polypeptid zum Nachweisen einer Hepatitis-E-Virus-Infektion nach dem Verfahren zum Nachweisen einer Hepatitis-E-Virus-Infektion nach Anspruch 1, das ein Dimer bestehend aus einem beliebigen Polypeptid ausgewählt aus der Gruppe bestehend aus den folgenden (3a) bis (3c) und einem beliebigen Polypeptid ausgewählt aus der Gruppe bestehend aus den folgenden (1a) bis (1c), (2a) bis (2c) und (4a) bis (4c) ist:
(1a) ein Polypeptid bestehend aus einer Aminosäuresequenz, die Aminosäuren an Positionen 28 bis 175 der in SEQ ID NO: 1 angegebenen Aminosäuresequenz enthält und die eine Gesamtlänge von 200 oder weniger Resten aufweist, die in der in SEQ ID NO: 1 angegebenen Aminosäuresequenz enthalten sind;
(1b) ein Polypeptid bestehend aus einer Aminosäuresequenz, bei der eine oder mehrere Aminosäuren in der Aminosäuresequenz substituiert, deletiert, eingefügt und/oder hinzugefügt sind, die Aminosäuren an Positionen 28 bis 175 der in SEQ ID NO: 1 angegebenen Aminosäuresequenz enthält und die eine Gesamtlänge von 200 oder weniger Resten aufweist, die in der in SEQ ID NO: 1 angegebenen Aminosäuresequenz enthalten sind;
(1c) ein Polypeptid bestehend aus einer Aminosäuresequenz, die eine Homologie von 80 % oder mehr mit bzw. zu der Aminosäuresequenz aufweist, die Aminosäuren an Positionen 28 bis 175 der in SEQ ID NO: 1 angegebenen Aminosäuresequenz enthält und die eine Gesamtlänge von 200 oder weniger Resten aufweist, die in der in SEQ ID NO: 1 angegebenen Aminosäuresequenz enthalten sind;
(2a) ein Polypeptid bestehend aus einer Aminosäuresequenz, die Aminosäuren an Positionen 28 bis 175 der in SEQ ID NO: 2 angegebenen Aminosäuresequenz enthält und die eine Gesamtlänge von 200 oder weniger Resten aufweist, die in der in SEQ ID NO: 2 angegebenen Aminosäuresequenz enthalten sind;
(2b) ein Polypeptid bestehend aus einer Aminosäuresequenz, bei der eine oder mehrere Aminosäuren in der Aminosäuresequenz substituiert, deletiert, eingefügt und/oder hinzugefügt sind, die Aminosäuren an Positionen 28 bis 175 der in SEQ ID NO: 2 angegebenen Aminosäuresequenz enthält und die eine Gesamtlänge von 200 oder weniger Resten aufweist, die in der in SEQ ID NO: 2 angegebenen Aminosäuresequenz enthalten sind;
(2c) ein Polypeptid bestehend aus einer Aminosäuresequenz, die eine Homologie von 80 % oder mehr mit bzw. zu der Aminosäuresequenz aufweist, die Aminosäuren an Positionen 28 bis 175 der in SEQ ID NO: 2 angegebenen Aminosäuresequenz enthält und die eine Gesamtlänge von 200 oder weniger Resten aufweist, die in der in SEQ ID NO: 2 angegebenen Aminosäuresequenz enthalten sind;
(3a) ein Polypeptid bestehend aus einer Aminosäuresequenz, die Aminosäuren an Positionen 28 bis 175 der in SEQ ID NO: 3 angegebenen Aminosäuresequenz enthält und die eine Gesamtlänge von 200 oder weniger Resten aufweist, die in der in SEQ ID NO: 3 angegebenen Aminosäuresequenz enthalten sind;
(3b) ein Polypeptid bestehend aus einer Aminosäuresequenz, bei der eine oder mehrere Aminosäuren in der Aminosäuresequenz substituiert, deletiert, eingefügt und/oder hinzugefügt sind, die Aminosäuren an Positionen 28 bis 175 der in SEQ ID NO: 3 angegebenen Aminosäuresequenz enthält und die eine Gesamtlänge von 200 oder weniger Resten aufweist, die in der in SEQ ID NO: 3 angegebenen Aminosäuresequenz enthalten sind;
(3c) ein Polypeptid bestehend aus einer Aminosäuresequenz, die eine Homologie von 80 % oder mehr mit bzw. zu der Aminosäuresequenz aufweist, die Aminosäuren an Positionen 28 bis 175 der in SEQ ID NO: 3 angegebenen Aminosäuresequenz enthält und die eine Gesamtlänge von 200 oder weniger Resten aufweist, die in der in SEQ ID NO: 3 angegebenen Aminosäuresequenz enthalten sind;
(4a) ein Polypeptid bestehend aus einer Aminosäuresequenz, die Aminosäuren an Positionen 28 bis 175 der in SEQ ID NO: 4 angegebenen Aminosäuresequenz enthält und die eine Gesamtlänge von 200 oder weniger Resten aufweist, die in der in SEQ ID NO: 4 angegebenen Aminosäuresequenz enthalten sind;
(4b) ein Polypeptid bestehend aus einer Aminosäuresequenz, bei der eine oder mehrere Aminosäuren in der Aminosäuresequenz substituiert, deletiert, eingefügt und/oder hinzugefügt sind, die Aminosäuren an Positionen 28 bis 175 der in SEQ ID NO: 4 angegebenen Aminosäuresequenz enthält und die eine Gesamtlänge von 200 oder weniger Resten aufweist, die in der in SEQ ID NO: 4 angegebenen Aminosäuresequenz enthalten sind; und
(4c) ein Polypeptid bestehend aus einer Aminosäuresequenz, die eine Homologie von 80 % oder mehr mit bzw. zu der Aminosäuresequenz aufweist, die Aminosäuren an Positionen 28 bis 175 der in SEQ ID NO: 4 angegebenen Aminosäuresequenz enthält und die eine Gesamtlänge von 200 oder weniger Resten aufweist, die in der in SEQ ID NO: 4 angegebenen Aminosäuresequenz enthalten sind.

5. Antigen-Polypeptid nach Anspruch 4, das ein markierter Körper ist, der mit einer Markierungssubstanz markiert ist.

6. Kit zum Nachweisen einer Hepatitis-E-Virus-Infektion nach dem Verfahren zum Nachweisen einer Hepatitis-E-Virus-Infektion nach Anspruch 1, umfassend das Antigen-Polypeptid nach Anspruch 4 oder 5.

7. Kit zum Nachweisen einer Hepatitis-E-Virus-Infektion nach Anspruch 6, ferner umfassend einen Fangkörper, der einen wasserunlöslichen Träger und ein Probenmolekül umfasst, das in der Lage ist, an den Anti-Hepatitis-E-Virus-Antikörper zu binden.

## Revendications

1. Procédé de détection d'une infection par le virus de l'hépatite E, comprenant une étape de détection consistant à détecter un anticorps anti-virus de l'hépatite E dans un échantillon en utilisant, en tant que polypeptide antigénique, un dimère constitué de tout polypeptide quelconque sélectionné parmi le groupe constitué des polypeptides (3a) à (3c) suivants et de tout polypeptide quelconque sélectionné parmi le groupe constitué des polypeptides (1a) à (1c), (2a) à (2c) et (4a) à (4c) suivants :
(1a) un polypeptide constitué d'une séquence d'acides aminés qui contient des acides aminés situés à la position 28 à 175 dans la séquence d'acides aminés établie dans SEQ ID N° : 1 et qui a une longueur entière de 200 résidus ou moins qui sont contenus dans la séquence d'acides aminés établie dans SEQ ID N° : 1 ;
(1b) un polypeptide constitué d'une séquence d'acides aminés dans laquelle un ou plusieurs acides aminés sont substitués, délétés, insérés et/ou ajoutés dans la séquence d'acides aminés qui contient des acides aminés situés à la position 28 à 175 dans la séquence d'acides aminés établie dans SEQ ID N° : 1 et qui a une longueur entière de 200 résidus ou moins qui sont contenus dans la séquence d'acides aminés établie dans SEQ ID N° : 1 ;
(1c) un polypeptide constitué d'une séquence d'acides aminés ayant 80 % ou plus d'homologie avec la séquence d'acides aminés qui contient des acides aminés situés à la position 28 à 175 dans la séquence d'acides aminés établie dans SEQ ID N° : 1 et qui a une longueur entière de 200 résidus ou moins qui sont contenus dans la séquence d'acides aminés établie dans SEQ ID N° : 1 ;
(2a) un polypeptide constitué d'une séquence d'acides aminés qui contient des acides aminés situés à la position 28 à 175 dans la séquence d'acides aminés établie dans SEQ ID N° : 2 et qui a une longueur entière de 200 résidus ou moins qui sont contenus dans la séquence d'acides aminés établie dans SEQ ID N° : 2 ;
(2b) un polypeptide constitué d'une séquence d'acides aminés dans laquelle un ou plusieurs acides aminés sont substitués, délétés, insérés et/ou ajoutés dans la séquence d'acides aminés qui contient des acides aminés situés à la position 28 à 175 dans la séquence d'acides aminés établie dans SEQ ID N° : 2 et qui a une longueur entière de 200 résidus ou moins qui sont contenus dans la séquence d'acides aminés établie dans SEQ ID N° : 2 ;
(2c) un polypeptide constitué d'une séquence d'acides aminés ayant 80 % ou plus d'homologie avec la séquence d'acides aminés qui contient des acides aminés situés à la position 28 à 175 dans la séquence d'acides aminés établie dans SEQ ID N° : 2 et qui a une longueur entière de 200 résidus ou moins qui sont contenus dans la séquence d'acides aminés établie dans SEQ ID N° : 2 ;
(3a) un polypeptide constitué d'une séquence d'acides aminés qui contient des acides aminés situés à la position 28 à 175 dans la séquence d'acides aminés établie dans SEQ ID N° : 3 et qui a une longueur entière de 200 résidus ou moins qui sont contenus dans la séquence d'acides aminés établie dans SEQ ID N° : 3 ;
(3b) un polypeptide constitué d'une séquence d'acides aminés dans laquelle un ou plusieurs acides aminés sont substitués, délétés, insérés et/ou ajoutés dans la séquence d'acides aminés qui contient des acides aminés situés à la position 28 à 175 dans la séquence d'acides aminés établie dans SEQ ID N° : 3 et qui a une longueur entière de 200 résidus ou moins qui sont contenus dans la séquence d'acides aminés établie dans SEQ ID N° : 3 ;
(3c) un polypeptide constitué d'une séquence d'acides aminés ayant 80 % ou plus d'homologie avec la séquence d'acides aminés qui contient des acides aminés situés à la position 28 à 175 dans la séquence d'acides aminés établie dans SEQ ID N° : 3 et qui a une longueur entière de 200 résidus ou moins qui sont contenus dans la séquence d'acides aminés établie dans SEQ ID N° : 3 ;
(4a) un polypeptide constitué d'une séquence d'acides aminés qui contient des acides aminés situés à la position 28 à 175 dans la séquence d'acides aminés établie dans SEQ ID N° : 4 et qui a une longueur entière de 200 résidus ou moins qui sont contenus dans la séquence d'acides aminés établie dans SEQ ID N° : 4 ;
(4b) un polypeptide constitué d'une séquence d'acides aminés dans laquelle un ou plusieurs acides aminés sont substitués, délétés, insérés et/ou ajoutés dans la séquence d'acides aminés qui contient des acides aminés situés à la position 28 à 175 dans la séquence d'acides aminés établie dans SEQ ID N° : 4 et qui a une longueur entière de 200 résidus ou moins qui sont contenus dans la séquence d'acides aminés établie dans SEQ ID N° : 4 ; et
(4c) un polypeptide constitué d'une séquence d'acides aminés ayant 80 % ou plus d'homologie avec la séquence d'acides aminés qui contient des acides aminés situés à la position 28 à 175 dans la séquence d'acides aminés établie dans SEQ ID N° : 4 et qui a une longueur entière de 200 résidus ou moins qui sont contenus dans la séquence d'acides aminés établie dans SEQ ID N° : 4.

2. Procédé de détection d'une infection par le virus de l'hépatite E selon la revendication 1, dans lequel un isotype de l'anticorps anti-virus de l'hépatite E est IgM.

3. Procédé de détection d'une infection par le virus de l'hépatite E selon la revendication 1, dans lequel
l'étape de détection comprend une étape consistant à mettre l'échantillon en contact avec un corps de capture et un corps marqué, et
le corps de capture comprend un support insoluble dans l'eau et une molécule de sonde capable de se lier à l'anticorps anti-virus de l'hépatite E, et
le corps marqué comprend une substance de marquage et le polypeptide antigénique.

4. Polypeptide antigénique pour détecter une infection par le virus de l'hépatite E conformément au procédé de détection d'une infection par le virus de l'hépatite E selon la revendication 1, qui est un dimère constitué de tout polypeptide quelconque sélectionné parmi le groupe constitué des polypeptides (3a) à (3c) suivants et de tout polypeptide quelconque sélectionné parmi le groupe constitué des polypeptides (1a) à (1c), (2a) à (2c) et (4a) à (4c) suivants :
(1a) un polypeptide constitué d'une séquence d'acides aminés qui contient des acides aminés situés à la position 28 à 175 dans la séquence d'acides aminés établie dans SEQ ID N° : 1 et qui a une longueur entière de 200 résidus ou moins qui sont contenus dans la séquence d'acides aminés établie dans SEQ ID N° : 1 ;
(1b) un polypeptide constitué d'une séquence d'acides aminés dans laquelle un ou plusieurs acides aminés sont substitués, délétés, insérés et/ou ajoutés dans la séquence d'acides aminés qui contient des acides aminés situés à la position 28 à 175 dans la séquence d'acides aminés établie dans SEQ ID N° : 1 et qui a une longueur entière de 200 résidus ou moins qui sont contenus dans la séquence d'acides aminés établie dans SEQ ID N° : 1 ;
(1c) un polypeptide constitué d'une séquence d'acides aminés ayant 80 % ou plus d'homologie avec la séquence d'acides aminés qui contient des acides aminés situés à la position 28 à 175 dans la séquence d'acides aminés établie dans SEQ ID N° : 1 et qui a une longueur entière de 200 résidus ou moins qui sont contenus dans la séquence d'acides aminés établie dans SEQ ID N° : 1 ;
(2a) un polypeptide constitué d'une séquence d'acides aminés qui contient des acides aminés situés à la position 28 à 175 dans la séquence d'acides aminés établie dans SEQ ID N° : 2 et qui a une longueur entière de 200 résidus ou moins qui sont contenus dans la séquence d'acides aminés établie dans SEQ ID N° : 2 ;
(2b) un polypeptide constitué d'une séquence d'acides aminés dans laquelle un ou plusieurs acides aminés sont substitués, délétés, insérés et/ou ajoutés dans la séquence d'acides aminés qui contient des acides aminés situés à la position 28 à 175 dans la séquence d'acides aminés établie dans SEQ ID N° : 2 et qui a une longueur entière de 200 résidus ou moins qui sont contenus dans la séquence d'acides aminés établie dans SEQ ID N° : 2 ;
(2c) un polypeptide constitué d'une séquence d'acides aminés ayant 80 % ou plus d'homologie avec la séquence d'acides aminés qui contient des acides aminés situés à la position 28 à 175 dans la séquence d'acides aminés établie dans SEQ ID N° : 2 et qui a une longueur entière de 200 résidus ou moins qui sont contenus dans la séquence d'acides aminés établie dans SEQ ID N° : 2 ;
(3a) un polypeptide constitué d'une séquence d'acides aminés qui contient des acides aminés situés à la position 28 à 175 dans la séquence d'acides aminés établie dans SEQ ID N° : 3 et qui a une longueur entière de 200 résidus ou moins qui sont contenus dans la séquence d'acides aminés établie dans SEQ ID N° : 3 ;
(3b) un polypeptide constitué d'une séquence d'acides aminés dans laquelle un ou plusieurs acides aminés sont substitués, délétés, insérés et/ou ajoutés dans la séquence d'acides aminés qui contient des acides aminés situés à la position 28 à 175 dans la séquence d'acides aminés établie dans SEQ ID N° : 3 et qui a une longueur entière de 200 résidus ou moins qui sont contenus dans la séquence d'acides aminés établie dans SEQ ID N° : 3 ;
(3c) un polypeptide constitué d'une séquence d'acides aminés ayant 80 % ou plus d'homologie avec la séquence d'acides aminés qui contient des acides aminés situés à la position 28 à 175 dans la séquence d'acides aminés établie dans SEQ ID N° : 3 et qui a une longueur entière de 200 résidus ou moins qui sont contenus dans la séquence d'acides aminés établie dans SEQ ID N° : 3 ;
(4a) un polypeptide constitué d'une séquence d'acides aminés qui contient des acides aminés situés à la position 28 à 175 dans la séquence d'acides aminés établie dans SEQ ID N° : 4 et qui a une longueur entière de 200 résidus ou moins qui sont contenus dans la séquence d'acides aminés établie dans SEQ ID N° : 4 ;
(4b) un polypeptide constitué d'une séquence d'acides aminés dans laquelle un ou plusieurs acides aminés sont substitués, délétés, insérés et/ou ajoutés dans la séquence d'acides aminés qui contient des acides aminés situés à la position 28 à 175 dans la séquence d'acides aminés établie dans SEQ ID N° : 4 et qui a une longueur entière de 200 résidus ou moins qui sont contenus dans la séquence d'acides aminés établie dans SEQ ID N° : 4 ; et
(4c) un polypeptide constitué d'une séquence d'acides aminés ayant 80 % ou plus d'homologie avec la séquence d'acides aminés qui contient des acides aminés situés à la position 28 à 175 dans la séquence d'acides aminés établie dans SEQ ID N° : 4 et qui a une longueur entière de 200 résidus ou moins qui sont contenus dans la séquence d'acides aminés établie dans SEQ ID N° : 4.

5. Polypeptide antigénique selon la revendication 4, qui est un corps marqué avec une substance de marquage.

6. Kit de détection d'une infection par le virus de l'hépatite E conformément au procédé de détection d'une infection par le virus de l'hépatite E selon la revendication 1, comprenant le polypeptide antigénique selon la revendication 4 ou 5.

7. Kit de détection d'une infection par le virus de l'hépatite E selon la revendication 6, comprenant en outre un corps de capture comprenant un support insoluble dans l'eau et une molécule de sonde capable de se lier à un anticorps anti-virus de l'hépatite E.
